(19) Europäisches Patentamt
European Patent Office
Office européen des brevets

(11) Veröffentlichungsnummer: 0 263 352
A1

# (12) EUROPÄISCHE PATENTANMELDUNG

(21) Anmeldenummer: 87113863.2

(22) Anmeldetag: 23.09.87

(51) Int. Cl.4: C07D 498/04 , A61K 31/535

(30) Priorität: 04.10.86 DE 3633861

(43) Veröffentlichungstag der Anmeldung:
13.04.88 Patentblatt 88/15

(84) Benannte Vertragsstaaten:
AT BE CH DE ES FR GB GR IT LI LU NL SE

(71) Anmelder: Dr. Karl Thomae GmbH
Postfach 1755
D-7950 Biberach (Riss)(DE)

(72) Erfinder: Narr, Berthold, Dr., Dipl.-Chem.
Obere Au 5
D-7950 Biberach 1(DE)
Erfinder: Hauel, Norbert, Dr., Dipl.-Chem.
Stresemannstrasse 40
D-7950 Biberach 1(DE)
Erfinder: Noll, Klaus, Dr., Dipl.-Chem.
Im Schönblick 3
D-7951 Warthausen(DE)
Erfinder: Heider, Joachim, Dr.Dipl.-Chem.
Am Hang 3
D-7951 Warthausen 1(DE)
Erfinder: Psiorz, Manfred, Dr.Dipl.-Chem.
Riedlinger Strasse 35
D-7950 Biberach 1(DE)
Erfinder: Bomhard, Andreas, Dr.Dipl.-Chem.
Dinglingerstrasse 9
D-7950 Biberach 1(DE)
Erfinder: Van Meel, Jacques, Dr.
Amriswilstrasse 7
D-7950 Biberach 1(DE)
Erfinder: Diederen, Willi, Dr.
Haldenstrasse 1a
D-7950 Biberach 1(DE)

## (54) Neue Imidazo-benzoxazinone, ihre Herstellung und diese Verbindungen enthaltende Arzneimittel.

(57) Die Erfindung betrifft neue Imidazo-benzoxazinone der Formel

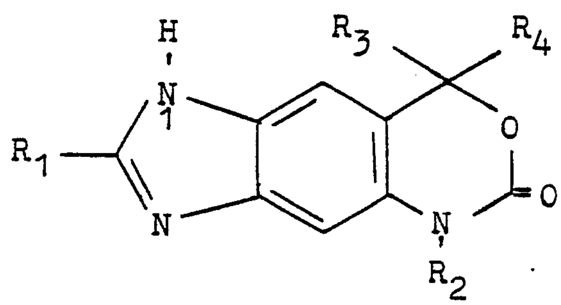

, (I)

in der

EP 0 263 352 A1

Xerox Copy Centre

$R_1$ eine gegebenenfalls durch eine Phenyl-oder Pyridylgruppe substituierte Alkylgruppe, eine gegebenenfalls durch eine Alkylgruppe substituierte Mercaptogruppe, eine Vinylengruppe, die endständig durch eine Phenyl-oder Pyridylgruppe substituiert ist, eine gegebenenfalls durch ein Halogenatom, eine Hydroxy-, Alkoxy-, Mercapto-, Alkylmercapto-, Alkylsulfinyl-, Alkylsulfonyl-, Cyano-, Amino-oder Nitrogruppe substituierte Phenylgruppe, wobei eine Hydroxyphenylgruppe zusätzlich durch eine oder zwei Alkylgruppen oder eine Aminophenylgruppe durch ein oder zwei Halogenatome substituiert sein kann, eine durch Halogenatome, Hydroxy-, Alkoxy-, Mercapto-, Alkylmercapto-, Alkylsulfinyl-oder Alkylsulfonylgruppen disubstituierte Phenylgruppe, wobei die Substituenten des Phenylkerns gleich oder verschieden sein können, einen über ein Kohlenstoffatom gebundenen 5-oder 6-gliedrigen heteroaromatischen Ring, welcher ein Sauerstoff-, Schwefel-oder Stickstoffatom, zwei oder drei Stickstoffatome oder ein Stickstoffatom und ein Sauerstoff-oder Schwefelatom enthält, an den zusätzlich über zwei benachbarte Kohlenstoffatome eine oder zwei 1,4-Butadienylgruppen gebunden sein können, wobei im Falle der Pyridine diese zusätzlich durch eine Amino-, Alkanoylamino-oder Morpholinogruppe oder durch zwei Halogenatome oder durch ein Halogen atom und eine Amino-oder Morpholinogruppe substituiert sein können, oder einen über einen Kohlenstoffatom gebundenen 5-bis 7-gliedrigen gesättigten Imino-, N-Alkyl-imino-oder N-Alkanoyl-imino-alkylenring in dem eine Methylengruppe in 4-Stellung durch eine Imino-, Alkylimino-oder Alkanoyliminogruppe oder eine $-CH_2CH_2-$Gruppe durch eine -NH-CO-Gruppe ersetzt sein kann, wobei die CO-Gruppe dieser -NH-CO-Gruppe mit dem bereits vorhandenen N-Atom verknüpft sein muß, und außerdem alle vorstehend erwähnten heteroaromatischen Ringe und gesättigten Ringe durch eine Alkylgruppe substituiert sein können,

$R_2$ ein Wasserstoffatom, eine gegebenenfalls ab Position 2 durch eine Hydroxy-oder Alkoxygruppe substituierte Alkylgruppe oder eine Phenylalkylgruppe,

$R_3$ und $R_4$, die gleich oder verschieden sein können, Wasserstoffatome oder Alkylgruppe bedeuten, deren 3H-Tautomere und deren Säureadditionssalze, insbesondere für die pharmazeutische Anwendung deren physiologisch verträgliche Säureadditionssalze mit anorganischen oder organischen Säuren, welche wertvolle pharmakologische Eigenschaften aufweisen, insbesondere antithrombotische und cardiovasculäre Eigenschaften.

Die neuen Verbindungen lassen sich nach bekannten Methoden herstellen.

# Neue Imidazo-benzoxazinone, ihre Herstellung und diese Verbindungen enthaltende Arzneimittel

Gegenstand der vorliegenden Erfindung sind neue Imidazobenzoxazinone der Formel

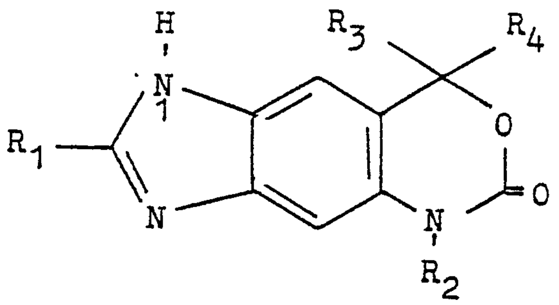

,(I)

deren 3H-Tautomere und deren Säureadditionssalze, insbesondere für die pharmazeutische Anwendung deren physiologisch verträgliche Säureadditionssalze mit anorganischen oder organischen Säuren, welche wertvolle pharmakologische Eigenschaften aufweisen, insbesondere antithrombotische und cardiovasculäre Eigenschaften, sowie diese Verbindungen enthaltende Arzneimittel und Verfahren zu ihrer Herstellung.

In der obigen Formel I bedeutet

$R_1$ eine gegebenenfalls durch eine Phenyl-oder Pyridylgruppe substituierte Alkylgruppe, eine Alkylgruppe mit 4 bis 6 Kohlenstoffatomen, eine gegebenenfalls durch eine Alkyl gruppe substituierte Mercaptogruppe, eine Vinylengruppe, die endständig durch eine Phenyl-oder Pyridylgruppe substituiert ist, eine gegebenenfalls durch ein Halogenatom, eine Hydroxy-, Alkoxy-, Mercapto-, Alkylmercapto-, Alkylsulfinyl-, Alkylsulfonyl-, Cyano-, Amino-oder Nitrogruppe substituierte Phenylgruppe, wobei eine Hydroxyphenylgruppe zusätzlich durch eine oder zwei Alkylgruppen mit jeweils 1 bis 4 Kohlenstoffatomen oder eine Aminophenylgruppe durch ein oder zwei Halogenatome substituiert sein kann, eine durch Halogenatome, Hydroxy-, Alkoxy-, Mercapto-, Alkylmercapto-, Alkylsulfinyl-oder Alkylsulfonylgruppen disubstituierte Phenylgruppe, wobei die Substituenten des Phenylkerns gleich oder verschieden sein können, einen über ein Kohlenstoffatom gebundenen 5-oder 6-gliedrigen heteroaromatischen Ring, welcher ein Sauerstoff-, Schwefel-oder Stickstoffatom, zwei oder drei Stickstoffatome oder ein Stickstoffatom und ein Sauerstoff-oder Schwefelatom enthält, an den zusätzlich über zwei benachbarte Kohlenstoffatome eine oder zwei 1,4-Butadienylgruppen gebunden sein können, wobei im Falle der Pyridine diese zusätzlich durch eine Amino-, Alkanoylamino-oder Morpholinogruppe oder durch zwei Halogenatome oder durch ein Halogenatom und eine Amino-oder Morpholinogruppe substituiert sein können, oder einen über einen Kohlenstoffatom gebundenen 5-bis 7-gliedrigen gesättigten Imino-, N-Alkyl-imino-oder N-Alkanoyl-imino-alkylenring in dem eine Methylengruppe in 4-Stellung durch eine Imino-, Alkylimino-oder Alkanoyliminogruppe oder eine $-CH_2CH_2$-Gruppe durch eine -NH-CO-Gruppe ersetzt sein kann, wobei die CO-Gruppe dieser -NH-CO-Gruppe mit dem bereits vorhandenen N-Atom verknüpft sein muß, und außerdem alle vorstehend erwähnten heteroaromatischen Ringe und gesättigten Ringe durch eine Alkylgruppe substituiert sein können,

$R_2$ ein Wasserstoffatom, eine gegebenenfalls ab Position 2 durch eine Hydroxy-oder Alkoxygruppe substituierte Alkylgruppe mit 1 bis 6 Kohlenstoffatomen oder eine Phenylalkylgruppe,

$R_3$ und $R_4$, die gleich oder verschieden sein können, Wasserstoffatome oder Alkylgruppe, wobei der Alkylteil aller vorstehend erwähnten Gruppen, sofern nichts anderes erwähnt wird, jeweils 1 bis 3 Kohlenstoffatome enthalten kann.

Für die bei der Definition der Reste $R_1$ bis $R_4$ eingangs erwähnten Bedeutungen kommt beispielsweise

für $R_1$ die Bedeutung der Methyl-, Äthyl-, n-Propyl-, Isopropyl-, n-Butyl-, Isobutyl-, tert.Butyl-, n-Pentyl-, 1-Methyl-n-butyl-, 2-Methyl-n-butyl-, 3-Methyl-n-butyl-, 1-Äthyl-n-propyl-, tert.Pentyl-, n-Hexyl-, Benzyl-, 1-Phenylethyl-, 2-Phenylethyl-, 3-Phenylpropyl-, Pyridylmethyl-, 1-Pyridylethyl-, 2-Pyridylethyl-, 2-Phenylethenyl-, 2-Pyridylethenyl-, Mercapto-, Methylmercapto-, Ethylmercapto-, n-Propylmercapto-, Isopropylmercapto-, Phenyl-, Fluorphenyl-, Difluorphenyl-, Chlorphenyl-, Dichlorphenyl-, Bromphenyl-, Dibromphenyl-, Hydroxyphenyl-, Dihydroxyphenyl-, Methoxyphenyl-, Dimethoxyphenyl-, Mercaptophenyl-, Bis-Mercaptophenyl-, Methylmercaptophenyl-, Bis-Methylmercaptophenyl-, Methylsulfinylphenyl-, Bis-Methylsulfinylphenyl-, Methylsulfonylphenyl-, Bis-Methylsulfonylphenyl-, Fluor-hydroxyphenyl-, Fluormethoxyphenyl-, Fluor-mercaptophenyl-, Fluor-methylmercaptophenyl-, Chlor-hydroxyphenyl-, Chlormethoxyphenyl-, Chlor-mercaptophenyl-, Chlor-methylmercaptophenyl-, Brom-hydroxyphenyl-, Brommethoxyphenyl-, Brom-mercaptophenyl-, Brom-methylmercaptophenyl-, Hydroxy-methoxyphenyl-, Hydroxymercaptophenyl-, Hydroxy-methylmercaptophenyl-, Methoxy-mercaptophenyl-, Methoxymethylmercaptophenyl-, Methoxy-methylsulfinylphenyl-, Cyanophenyl-, Aminophenyl-, Amino-dichlorphenyl-, Amino-dibromphenyl-, Nitrophenyl-, 4-Hydroxy-3,5-di-tert.butyl-phenyl-, Pyridyl-, Methylpyridyl-, Ethylpyridyl-, Isopropylpyridyl-, 2-Amino-pyridyl-5-, 2-Formylamino-pyridyl-5-, 2-Acetylamino-pyridyl-5-, 2-Propionylamino-pyridyl-5-, 2,6-Dichlor-pyridyl-4-, 2-Chlor-6-amino-pyridyl-4-, 2-Chlor-6-morpholino-pyridyl-4-, 2,6-Dichlor-pyridyl-3-, 2-Chlor-6-morpholino-pyridyl-3-, Pyrrolyl-2-, Pyrrolyl-3-, N-Methyl-pyrrolyl-2-, N-Ethyl-pyrrolyl-2-, N-Methyl-pyrrolyl-3-, N-Ethyl-pyrrolyl-3-, Furyl-2-, Furyl-3-, 5-Methyl-furyl-2-, 2-Methyl-furyl-3-, Pyrazinyl-2-, Pyrimidyl-2-, Pyrimidyl-4-, Pyrimidyl-5-, Benzo[b]furyl-2-, Benzo[b]furyl-3-, 7-Methyl-benzo[b]furyl-3-, Thienyl-2-, Thienyl-3-, 5-Methylthienyl-2-, 2-Methyl-thienyl-3-, 3-Methyl-thienyl-2-, Benzo-[b]thienyl-2-, Benzo[b]thienyl-3-, Benzo[b]thienyl-4-, Benzo[b]thienyl-5-, Benzo[b]thienyl-6-, Benzo[b]thienyl-7-, Pyrazolyl-3-, Imidazolyl-2-, Imidazolyl-4(5)-, 1-Methyl-imidazolyl-4-, Benzo[d]imidazolyl-2-, Oxazolyl-2-, Oxazolyl-4-, Oxazolyl-5-, 4-Methyl-oxazolyl-5-, Isoxazolyl-3-, 3-Methyl-isoxazolyl-5-, 5-Methyl-isoxazolyl-3-, Thiazolyl-2-, Thiazolyl-5-, 4-Methyl-thiazolyl-5-, Pyrazolyl-4-, Triazolyl-3-, Benzo[d]oxazolyl-2-, Benzo[d]-isoxazolyl-3-, Benzo[d]thiazolyl-2-, Benzo[d]isothiazolyl-3-, Benzo[d]pyrazolyl-3-, Indolyl-2-, Indolyl-3-, 5-Methyl-indolyl-3-, 7-Methyl-indolyl-3-, N-Methyl-indolyl-3-, Chinolyl-2-, Isochinolyl-1-, 2-Methyl-chinolyl-4-oder 7-Methyl-chinolyl-2-gruppe,

für $R_2$ die des Wasserstoffatoms, der Methyl-, Ethyl-, n-Propyl-, Isopropyl-, n-Butyl-, Isobutyl-, tert.Butyl-, n-Pentyl-, 1-Methyl-n-butyl-, 2-Methyl-n-butyl-, 3-Methyl-n-butyl-, 1-Äthyl-n-propyl-, tert.Pentyl-, n-Hexyl-, Benzyl-, 1-Phenylethyl-, 2-Phenylethyl-, 3-Phenylpropyl-, 2-Hydroxy-äthyl-, 2-Hydroxy-n-propyl-, 3-Hydroxy-n-propyl-, 1-Methyl-2-hydroxy-äthyl-, 2-Methoxy-äthyl-, 2-Äthoxy-äthyl-, 2-Methoxy-n-propyl-, 2-n-Propoxyn-propyl-, 3-Methoxy-n-propyl-, 3-Äthoxy-n-propyl-, 1-Methyl-2-methoxy-äthyl-oder 1-Methyl-2-isopropoxy-äthyl-gruppe,

für $R_3$ und $R_4$ jeweils die des Wasserstoffatoms, der Methyl-, Ethyl-, n-Propyl-oder Isopropylgruppe in Betracht.

Beispielsweise seien folgende Verbindungen erwähnt, die unter den Schutzumfang der vorliegenden Erfindung fallen und nicht in den Beispielen explicite erwähnt werden:

8,8-Dimethyl-5-(2-hydroxy-äthyl)-2-(4-pyridyl)-8-hydro-(5H)-imidazo[5,4-g][3,1]benzoxazin-6-on,

8,8-Dimethyl-5-(2-hydroxy-äthyl)-2-(3-pyridyl)-8-hydro-(5H)-imidazo[5,4-g][3,1]benzoxazin-6-on,

8,8-Dimethyl-2-(2-pyridyl)-8-hydro-(5H)-imidazo[5,4-g][3,1]benzoxazin-6-on,

2-(2-Pyridyl)-5,8,8-trimethyl-8-hydro-(5H)-imidazo[5,4-g][3,1]benzoxazin-6-on,

5-Äthyl-8,8-dimethyl-2-(2-pyridyl)-8-hydro-(5H)-imidazo[5,4-g][3,1]benzoxazin-6-on,

8,8-Dimethyl-5-(2-methoxy-äthyl)-2-(2-pyridyl)-8-hydro-(5H)-imidazo[5,4-g][3,1]benzoxazin-6-on,

8,8-Dimethyl-5-(2-hydroxy-äthyl)-2-(2-pyridyl)-8-hydro-(5H)-imidazo[5,4-g][3,1]benzoxazin-6-on,

8,8-Dimethyl-2-(4-hydroxyphenyl)-8-hydro-(5H)-imidazo[5,4-g][3,1]benzoxazin-6-on,

5-Äthyl-8,8-dimethyl-2-(4-hydroxyphenyl)-8-hydro-(5H)-imidazo[5,4-g][3,1]benzoxazin-6-on,

8,8-Dimethyl-2-(4-hydroxyphenyl)-5-(2-methoxy-äthyl)-8-hydro-(5H)-imidazo[5,4-g][3,1]benzoxazin-6-on,

8,8-Dimethyl-5-(2-hydroxy-äthyl)-2-(4-hydroxyphenyl)-8-hydro-(5H)-imidazo[5,4-g][3,1]benzoxazin-6-on,

8,8-Dimethyl-5-(2-methoxy-äthyl)-2-(4-methoxyphenyl)-8-hydro-(5H)-imidazo[5,4-g][3,1]benzoxazin-6-on,

8,8-Dimethyl-5-(2-hydroxy-äthyl)-2-(4-methoxyphenyl)-8-hydro-(5H)-imidazo[5,4-g][3,1]benzoxazin-6-on,

5-Äthyl-2-(4-pyridyl)-8-hydro-(5H)-imidazo[5,4-g][3,1]benzoxazin-6-on,

5-(2-Methoxy-äthyl)-2-(4-pyridyl)-8-hydro-(5H)-imidazo[5,4-g][3,1]benzoxazin-6-on,

5-(2-Hydroxy-äthyl)-2-(4-pyridyl)-8-hydro-(5H)-imidazo[5,4-g][3,1]benzoxazin-6-on,

2-(3-Pyridyl)-8-hydro-(5H)-imidazo[5,4-g][3,1]benzoxazin-6-on,

5-Äthyl-2-(3-pyridyl)-8-hydro-(5H)-imidazo[5,4-g][3,1]benzoxazin-6-on,

5-(2-Methoxy-äthyl)-2-(3-pyridyl)-8-hydro-(5H)-imidazo[5,4-g][3,1]benzoxazin-6-on,

5-(2-Hydroxy-äthyl)-2-(3-pyridyl)-8-hydro-(5H)-imidazo[5,4-g][3,1]benzoxazin-6-on,

2-(2-Pyridyl)-8-hydro-(5H)-imidazo[5,4-g][3,1]benzoxazin-6-on,

5-Methyl-2-(2-pyridyl)-8-hydro-(5H)-imidazo[5,4-g][3,1]benzoxazin-6-on,

5-Äthyl-2-(2-pyridyl)-8-hydro-(5H)-imidazo[5,4-g][3,1]benzoxazin-6-on,

5-(2-Methoxy-äthyl)-2-(2-pyridyl)-8-hydro-(5H)-imidazo[5,4-g][3,1]benzoxazin-6-on,

5-(2-Hydroxy-äthyl)-2-(2-pyridyl)-8-hydro-(5H)-imidazo[5,4-g][3,1]benzoxazin-6-on,

2-(4-Hydroxyphenyl)-8-hydro-(5H)-imidazo[5,4-g][3,1]benzoxazin-6-on,

2-(4-Hydroxyphenyl)-5-methyl-8-hydro-(5H)-imidazo[5,4-g][3,1]benzoxazin-6-on,

5-Äthyl-2-(4-hydroxyphenyl)-8-hydro-(5H)-imidazo[5,4-g][3,1]benzoxazin-6-on,

2-(4-Hydroxyphenyl)-5-(2-methoxy-äthyl)-8-hydro-(5H)-imidazo[5,4-g][3,1]benzoxazin-6-on,

5-(2-Hydroxy-äthyl)-2-(4-hydroxyphenyl)-8-hydro-(5H)-imidazo[5,4-g][3,1]benzoxazin-6-on,

2-(4-Methoxyphenyl)-8-hydro-(5H)-imidazo[5,4-g][3,1]benzoxazin-6-on,

5-Äthyl-2-(4-methoxyphenyl)-8-hydro-(5H)-imidazo[5,4-g][3,1]benzoxazin-6-on,

5-(2-Methoxy-äthyl)-2-(4-methoxyphenyl)-8-hydro-(5H)-imidazo[5,4-g][3,1]benzoxazin-6-on,

5-(2-Hydroxy-äthyl)-2-(4-methoxyphenyl)-8-hydro-(5H)-imidazo[5,4-g][3,1]benzoxazin-6-on,

8-Methyl-2-(4-pyridyl)-8-hydro-(5H)-imidazo[5,4-g][3,1]benzoxazin-6-on,

5,8-Dimethyl-2-(4-pyridyl)-8-hydro-(5H)-imidazo[5,4-g][3,1]benzoxazin-6-on,

5-Äthyl-8-methyl-2-(4-pyridyl)-8-hydro-(5H)-imidazo[5,4-g][3,1]benzoxazin-6-on,

5-(2-Methoxy-äthyl)-8-methyl-2-(4-pyridyl)-8-hydro-(5H)-imidazo[5,4-g][3,1]benzoxazin-6-on,

5-(2-Hydroxy-äthyl)-8-methyl-2-(4-pyridyl)-8-hydro-(5H)-imidazo[5,4-g][3,1]benzoxazin-6-on,

8-Methyl-2-(3-pyridyl)-8-hydro-(5H)-imidazo[5,4-g][3,1]benzoxazin-6-on,

5,8-Dimethyl-2-(3-pyridyl)-8-hydro-(5H)-imidazo[5,4-g][3,1]benzoxazin-6-on,

5-Äthyl-8-methyl-2-(3-pyridyl)-8-hydro-(5H)-imidazo[5,4-g][3,1]benzoxazin-6-on,

5-(2-Methoxy-äthyl)-8-methyl-2-(3-pyridyl)-8-hydro-(5H)-imidazo[5,4-g][3,1]benzoxazin-6-on,

5-(2-Hydroxy-äthyl)-8-methyl-2-(3-pyridyl)-8-hydro-(5H)-imidazo[5,4-g][3,1]benzoxazin-6-on.

8-Methyl-2-(2-pyridyl)-8-hydro-(5H)-imidazo[5,4-g][3,1]benzoxazin-6-on,

5,8-Dimethyl-2-(2-pyridyl)-8-hydro-(5H)-imidazo[5,4-g][3,1]benzoxazin-6-on,

5-Äthyl-8-methyl-2-(2-pyridyl)-8-hydro-(5H)-imidazo[5,4-g][3,1]benzoxazin-6-on,

5-(2-Methoxy-äthyl)-8-methyl-2-(2-pyridyl)-8-hydro-(5H)-imidazo[5,4-g][3,1]benzoxazin-6-on,

5-(2-Hydroxy-äthyl)-8-methyl-2-(2-pyridyl)-8-hydro-(5H)-imidazo[5,4-g][3,1]benzoxazin-6-on,

2-(4-Hydroxyphenyl)-8-methyl-8-hydro-(5H)-imidazo[5,4-g][3,1]benzoxazin-6-on,

5,8-Dimethyl-2-(4-hydroxyphenyl)-8-hydro-(5H)-imidazo[5,4-g][3,1]benzoxazin-6-on,

5-Äthyl-2-(4-hydroxyphenyl)-8-methyl-8-hydro-(5H)-imidazo[5,4-g][3,1]benzoxazin-6-on,

2-(4-Hydroxyphenyl)-5-(2-methoxy-äthyl)-8-methyl-8-hydro-(5H)-imidazo[5.4-g][3,1]benzoxazin-6-on,

5-(2-Hydroxy-äthyl)-2-(4-hydroxyphenyl)-8-methyl-8-hydro-(5H)-imidazo[5,4-g][3,1]benzoxazin-6-on,

2-(4-Methoxyphenyl)-8-methyl-8-hydro-(5H)-imidazo[5,4-g][3,1]benzoxazin-6-on,

5,8-Dimethyl-2-(4-methoxyphenyl)-8-hydro-(5H)-imidazo[5,4-g][3,1]benzoxazin-6-on,

5-Äthyl-2-(4-methoxyphenyl)-8-methyl-8-hydro-(5H)-imidazo[5,4-g][3,1]benzoxazin-6-on,

5-(2-Methoxy-äthyl)-2-(4-methoxyphenyl)-8-methyl-8-hydro-(5H)-imidazo[5,4-g][3,1]benzoxazin-6-on und

5-(2-Hydroxy-äthyl)-2-(4-methoxyphenyl)-8-methyl-8-hydro-(5H)-imidazo[5,4-g][3,1]benzoxazin-6-on,

deren 3H-Tautomere und deren Säureadditionssalze.

Bevorzugte Verbindungen der obigen Formel I sind jedoch diejenigen. in denen

$R_1$ eine gegebenenfalls durch eine Phenyl-oder Pyridylgruppe substituierte Alkylgruppe mit 1 oder 2 Kohlenstoffatomen, eine Alkylgruppe mit 3 bis 5 Kohlenstoffatomen, eine gegebenenfalls durch eine Methylgruppe substituierte Mercaptogruppe, eine Vinylengruppe, die endständig durch eine Phenyl-oder Pyridylgruppe substituiert ist, eine gegebenenfalls durch eine Hydroxy-, Methoxy-, Methylmercapto-, Methylsulfinyl-, Methylsulfonyl-, Cyano-oder Nitrogruppe substituierte Phenylgruppe, eine Dimethoxyphenyl-, 3,5-Di-tert.butyl-4-hydroxy-phenyl-, 4-Amino-3,5-dihalogen-phenyl-, Pyridyl-, Piperidinyl-, Morpholino-pyridyl-, Chinolyl-, Furanyl-, Thienyl-oder Pyrazinylgruppe,

$R_2$ ein Wasserstoffatom, eine Methyl-, Benzyl-, Äthyl-, n-Propyl-, Isopropyl-, 2-Hydroxy-äthyl-oder 2-Methoxy-äthylgruppe,

$R_3$ und $R_4$, die gleich oder verschieden sein können, Wasserstoffatome, Methyl-oder Ethylgruppen bedeuten, deren 3H-Tautomere und deren Säureadditionssalze.

Besonders bevorzugt sind jedoch die Verbindungen der Formel I, in denen

$R_1$ eine Alkylgruppe mit 1 bis 3 Kohlenstoffatomen oder eine Pyridyl-, Pyrazinyl-, Furyl-oder Thienylgruppe,

$R_2$ ein Wasserstoffatom, eine Methyl-oder Ethylgruppe,

$R_3$ und $R_4$ jeweils eine Methylgruppe bedeuten, 3H-Tautomere und deren Säureadditionssalze.

Erfindungsgemäß erhält man die neuen Verbindungen nach folgenden Verfahren:

a) Cyclisierung einer Verbindung der Formel

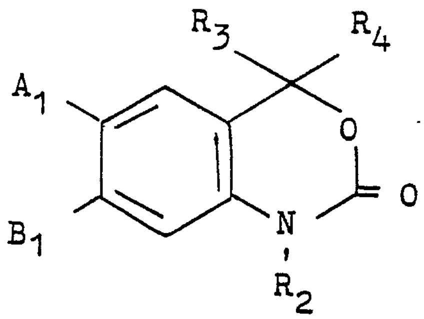

,(II)

in der
$R_2$ bis $R_4$ wie eingangs definiert sind,

einer der Reste $A_1$ oder $B_1$ eine $NH_2$-Gruppe und der andere der Reste $A_1$ oder $B_1$ eine

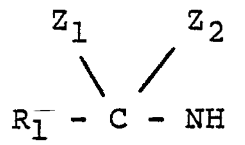

- Gruppe

bedeuten, wobei

$R_1$ wie eingangs definiert ist,

$Z_1$ und $Z_2$, die gleich oder verschieden sein können, nukleophile Austrittsgruppen wie gegebenenfalls substituierte Amino-, Alkoxy-, Phenylalkoxy-, Phenoxy-, Alkylmercapto-, Phenylalkylmercapto-oder Phenylthiogruppen oder

$Z_1$ und $Z_2$ zusammen ein Sauerstoff-oder Schwefelatom, eine gegebenenfalls durch eine Alkyl-oder Phenylalkylgruppe substituierte Iminogruppe, wobei die vorstehend erwähnten Alkylteile jeweils 1 bis 3 Kohlenstoffatome enthalten können, oder eine Alkylendioxigruppe mit 2 oder 3 Kohlenstoffatomen bedeuten.

Für $Z_1$ und $Z_2$ kommt beispielsweise jeweils die der Methoxy-, Ethoxy-, Propoxy-, Benzyloxy-, Methylmercapto-, Ethylmercapto-, Propylmercapto-, Phenylmercapto-oder Benzylmercaptogruppe oder

für $Z_1$ und $Z_2$ zusammen mit dem Kohlenstoffatom eine Carbonyl-oder Thiocarbonylgruppe in Betracht.

Die Umsetzung wird zweckmäßigerweise in einem Lösungsmittel oder Lösungsmittelgemisch wie Ethanol, Isopropanol, Eisessig, Tetrahydrofuran, Dioxan, Benzol, Toluol, Xylol, Chlorbenzol, Glycol, Glycolmonomethyläther, Glycoldimethyläther, Diäthylenglycoldimethylether, Dimethylformamid, Tetralin oder Sulfolan gegebenenfalls in Gegenwart eines Kondensationsmittels wie Phosphoroxychlorid, Thionylchlorid, Sulfurylchlorid, Salzsäure, Schwefelsäure, Phosphorsäure, Polyphosphorsäure, p-Toluolsulfonsäure, Eisessig, Essigsäureanhydrid, N,N'-Dicyclohexyl-carbodiimid, Carbonyldiimidazol, Kaliummethylat oder Kalium-tert.butylat bei Temperaturen zwischen 0 und 300°C, vorzugsweise jedoch bei der Siedetemperatur des Reaktionsgemisches, z.B. bei Temperaturen zwischen 50 und 285°C, durchgeführt. Die Umsetzung kann jedoch auch in der Schmelze durchgeführt werden.

Besonders vorteilhaft wird die Umsetzung jedoch in der Weise durchgeführt, daß eine Ausgangsverbindung der Formel II im Reaktionsgemisch durch Reduktion einer entsprechenden Nitroverbindung der Formel

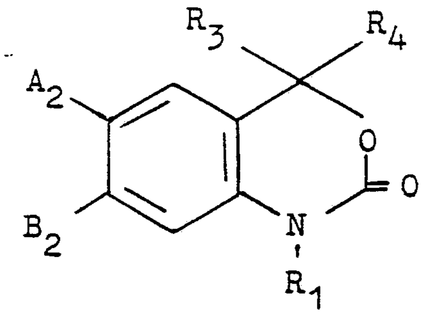

,(III)

in der
$R_2$, $R_3$ und $R_4$ wie eingangs definiert sind,

einer der Reste $A_2$ oder $B_2$ eine Nitrogruppe und

der andere der Reste $A_2$ und $B_2$ eine Gruppe der Formel

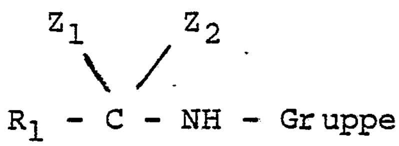

darstellt, in welcher

$R_1$, $Z_1$ und $Z_2$ wie eingangs definiert sind, oder

durch Umsetzung einer Verbindung der Formel

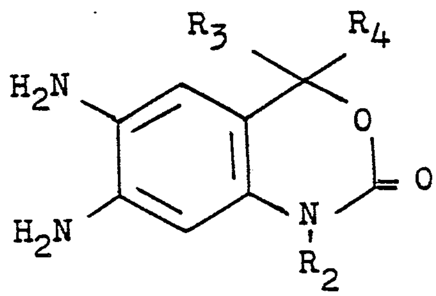

,(IV)

in der
$R_2$, $R_3$ und $R_4$ wie eingangs definiert sind, mit einem reaktionsfähigen Derivat einer Carbonsäure der Formel

$R_1$-COOH ,(V)

in der
$R_1$ wie eingangs definiert ist, wie dessen Nitrils, Anhydrids, Esters, Thioesters, Orthoesters, Halogenids, Methojodids, Amids oder Thioamids, hergestellt wird.

Die Reduktion einer Nitrogruppe wird vorzugsweise in einem geeigneten Lösungsmittel wie Eisessig, Wasser, Äthanol oder Wasser/Eisessig zweckmäßigerweise mit nascierendem Wasser stoff, z.B. in Gegenwart von Zink/Eisessig, Zinn/Salzsäure oder Zinn(II)chlorid/Salzsäure, oder mit katalytisch angeregtem Wasserstoff, z.B. mit Wasserstoff in Gegenwart von Platin, Palladium oder Raney-Nickel, bei Temperaturen zwischen 0 und 150°C, vorzugsweise bei Temperaturen zwischen 20 und 125°C, durchgeführt. Hierbei kann eine im Rest $R_1$ vorhandene Doppelbindung mitreduziert werden.

Die Acylierung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

b) Umsetzung einer Verbindung der Formel

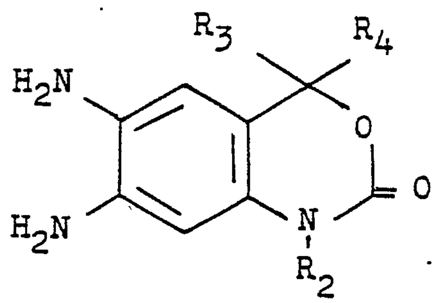

,(IV)

in der

$R_2$, $R_3$ und $R_4$ wie eingangs definiert sind, mit einem Aldehyd der Formel

$R_1$-CHO ,(VI)

in der

$R_1$ wie eingangs definiert ist, und anschließende Oxidation.

Die Umsetzung einer Verbindung der Formel IV mit einem Aldehyd der Formel VI wird zweckmäßigerweise in einem geeigneten Lösungsmittel wie Methanol, Ethanol, Eisessig oder Ethlyenchlorid bei Temperaturen zwischen 20 und 100°C durchgeführt. Die anschließende Oxidation wird mit einem Oxidationsmittel wie Sauerstoff, Wasserstoffperoxid oder einer Persäure wie m-Chlor-perbenzoesäure bei Temperaturen zwischen 20 und 100°C, vorzugsweise bei Temperaturen zwischen 40 und 80°C, durchgeführt.

Erhält man erfindungsgemäß eine Verbindung der Formel I, in der $R_2$ ein Wasserstoffatom darstellt und/oder $R_1$ eine Hydroxy-oder Mercaptogruppe enthält, so kann diese mittels Alkylierung in die entsprechende Alkylverbindung übergeführt werden und/oder

eine Verbindung der Formel I, in der $R_1$ eine endständig durch eine Phenyl-oder Pyridylgruppe substituierte Vinylengruppe darstellt, so kann diese mittels katalytischer Hydrierung in eine entsprechende Verbindung der Formel I, in der $R_1$ eine endständig durch eine Phenyl-oder Pyridylgruppe substituierte Ethylengruppe darstellt, übergeführt werden und/oder

eine Verbindung der Formel I, in der $R_2$ eine Benzylgruppe darstellt, so kann diese mittels katalytischer Hydrierung in eine entsprechende Verbindung der Formel I, in der $R_2$ ein Wasserstoff darstellt, übergeführt werden und/oder

eine Verbindung der Formel I, in der $R_1$ ein Pyridinring darstellt, so kann dieser mittels katalytischer Hydrierung in eine entsprechende Verbindung der Formel I, in der $R_1$ eine Piperidinylgruppe darstellt, übergeführt werden und/oder

eine Verbindung der Formel I, in der $R_1$ eine Alkoxyphenyl-oder Dialkoxyphenylgruppe darstellt, so kann diese mittels Etherspaltung in eine entsprechende Verbindung der Formel I, in der $R_1$ eine Hydroxyphenyl-

oder Dihydroxyphenylgruppe darstellt, übergeführt werden und/oder

eine Verbindung der Formel I, in der $R_1$ eine Alkylmercaptophenyl-oder Dialkylmercaptophenylgruppe darstellt, so kann diese mittels Oxidation in eine entsprechende Verbindung der Formel I, in der $R_1$ eine Alkylsulfinyl-, Alkylsulfonyl-, Dialkylsulfinyl-oder Dialkylsulfonyl-phenylgruppe darstellt, übergeführt werden.

Die nachträgliche Alkylierung wird in einem geeigneten Lösungsmittel wie Methanol, Ethanol, Diethylether, Aceton, Methylenchlorid, Tetrahydrofuran, Dioxan, Dimethylformamid oder Dimethylsulfoxid, gegebenenfalls in Gegenwart einer Base wie Natriumcarbonat, Kalium-tert.butylat, Triethylamin oder Pyridin, wobei die beiden letzteren gleichzeitig auch als Lösungsmittel dienen können, mit einem geeigneten Alkylierungsmittel wie Methyljodid, Dimethylsulfat, Ethylbromid, Diethylsulfat, n-Propylbromid, Isopropylbromid, Ethylenoxid, 2-Hy droxy-ethylbromid oder Formaldehyd/Ameisensäure oder in Gegenwart von Natriumcyanborhyrid, falls eine entsprechende Carbonylverbindung eingesetzt wird, bei Temperaturen zwischen 0 und 100°C, durchgeführt.

Die nachträgliche katalytische Hydrierung wird vorzugsweise mit Wasserstoff in Gegenwart eines Hydrierungskatalysators wie Platin, Palladium/Kohle oder Raney-Nickel in einem geeigneten Lösungsmittel, z.B. in Methanol, Ethanol, Tetrahydrofuran, Dioxan, Eisessig, Dimethylformamid oder Essigsäureethylester, bei Temperaturen zwischen 20 und 100°C, vorzugsweise bei 20 bis 50°C, durchgeführt.

Die nachträgliche Etherspaltung wird zweckmäßigerweise in Gegenwart einer Säure wie Salzsäure, Schwefelsäure, Phosphorsäure oder Bortribromid in einem geeigneten Lösungsmittel wie Ethylenchlorid bei Temperaturen zwischen -10°C und der Siedetemperatur des verwendeten Lösungsmittels, vorzugsweise bei Temperaturen zwischen 0 und 50°C, durchgeführt.

Die nachträgliche Oxidation wird vorzugsweise in einem Lösungsmittel oder Lösungsmittelgemisch, z.B. in Wasser, Wasser/Pyridin, Methanol, Ethanol, Aceton, Ameisensäure, Eisessig, verdünnter Schwefelsäure oder Trifluoressigsäure, je nach dem verwendeten Oxidationsmittel zweckmäßigerweise bei Temperaturen zwischen -80 und 100°C, durchgeführt.

Zur Herstellung einer Alkylsulfinylverbindung der Formel I wird die Oxidation zweckmäßigerweise mit einem Äquivalent des verwendeten Oxidationsmittels durchgeführt, z.B. mit Wasserstoffperoxid in Eisessig oder Ameisensäure bei 0 bis 20°C oder in Aceton bei 0 bis 60°C, mit einer Persäure wie Perameisensäure oder m-Chlor-perbenzoesäure in Eisessig oder Trifluoressigsäure bei 0 bis 20°C, mit Natriummetaperjodat in wässrigem Methanol oder Ethanol bei 15 bis 25°C oder mit tert.Butyl-hypochlorit in Methanol bei -80 bis -30°C.

Zur Herstellung einer Alkylsulfonylverbindung der Formel I wird die Oxidation zweckmäßigerweise mit einem bzw. mit mehr Äquivalenten des verwendeten Oxidationsmittels durchgeführt, z.B. mit Wasserstoff in Eisessig oder in Ameisensäure bei 20 bis 100°C oder in Aceton bei 0 bis 60°C, mit einer Persäure wie Perameisensäure oder m-Chlor-perbenzoesäure in Eisessig, Trifluoressigsäure oder Chloroform bei 0 bis 50°C, oder mit Bromsäure oder Kaliumpermanganat in Eisessig, Wasser/Schwefelsäure oder in Aceton bei 0 bis 20°C. Enthält somit eine eingesetzte Verbindung der Formel I eine Alkylmercaptogruppe, so wird die nachträgliche Oxidation vorzugsweise mit zwei oder mehr Äquivalenten des betreffenden Oxidationsmittels und ganz entsprechend mit mindestens einem Äquivalent durchgeführt, falls eine eingesetzte Verbindung der Formel I eine Alkylsulfinylgruppe enthält.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis VI erhält man nach literaturbekannten Verfahren.

So erhält man beispielsweise 4,4-Dimethyl-6-isonicotinoylamino-7-nitro-4H-3,1-benzoxazin-2-on durch Umsetzung von Anthranilsäuremethylester mit Methylmagnesiumjodid in ätherischer Lösung zu o-Aminophenyldimethylcarbinol, welches durch Umsetzung mit Phosgen in einem Gemisch von Toluol und Chloroform als Lösungsmittel in Gegenwart von festem Kaliumcarbonat zu 4,4-Dimethyl-4H-3,1-benzoxazin-2-on zyklisiert wird. Durch anschließende Nitrierung mit rauchender Salpetersäure (d = 1,52) erhält man 4,4-Dimethyl-6-nitro-benzoxazin-2-on, welches durch katalytische Hydrierung in Gegenwart von Raney-Nickel in Dimethylformamid in 6-Amino-4,4-dimethyl-4H-3,1-benzoxazin-2-on übergeführt wird. Durch Einführung einer Acetyl-Schutzgruppe mittels Essigsäureanhydrid schafft man die Voraussetzung für eine weitere Nitrierung mit rauchender Salpetersäure (d = 1,52) in der 7-Position des Moleküls. Von dem so erhaltenen

6-Acetamido-4,4-dimethyl-7-nitro-4H-3,1-benzoxazin-2-on spaltet man durch Behandeln mit konz. Salzsäure in Isopropanol die Acetyl-Schutzgruppe wieder ab und führt das so erhaltene 6-Amino-4,4-dimethyl7-nitro-4H-3.1-benzoxazin-2-on durch Behandeln mit Isonicotinsäurechlorid in Pyridin als Lösungsmittel in 4,4-Dimethyl-6-isonicotinoylamido-7-nitro-4H-3,1-benzoxazin-2-on über.

Die neuen Verbindungen der Formel I, deren optisch aktiven Antipoden und deren Säureadditionssalze, insbesondere deren physiologisch verträgliche Säureadditionssalze mit anorganischen oder organischen Säuren weisen, wie bereits eingangs erwähnt, wertvolle pharmakologische Eigenschaften auf, insbesondere bei einer geringen Beeinflußung des Blutdruckes cardiovasculäre Wirkungen, nämlich eine cardiotonische, und eine antithrombotische Wirkung.

Beispielsweise wurden die Verbindungen

A = 8,8-Dimethyl-2-(4-pyridyl)-8-hydro-(5H)-imidazo[5,4-g][3,1]benzoxazin-6-on,
B = 5-Äthyl-8,8-dimethyl-2-(4-pyridyl)-8-hydro-(5H)-imidazo[5,4-g][3,1]-benzoxazin-6-on,
C = 8,8-Dimethyl-2-n-propyl-8-hydro-(5H)-imidazo[5,4-g][3,1]benzoxazin-6-on,
D = 5-Äthyl-8,8-dimethyl-2-(2-furyl)-8-hydro-(5H)-imidazo[5,4-g][3,1]benzoxazin-6-on,
E = 8,8-Dimethyl-2-(2-furyl)-8-hydro-(5H)-imidazo[5,4-g][3,1]benzoxazin-6-on und
F = 8,8-Dimethyl-2-(2-thienyl)-8-hydro-(58)-imidazo[5,4-g][3,1]benzoxazin-6-on auf ihre biologischen Eigenschaften wie folgt untersucht:

1. Bestimmung der blutdrucksenkenden und positiv inotropen Wirkung an der narkotisierten Katze:

Die Untersuchungen wurden an Katzen durchgeführt, die mit Pentobarbital-Natrium (40 mg/kg i.p. und 8 mg/kg/Stunde i.v.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter $dp/dt_{max}$ mittels eines Analogdifferenzierers gewonnen. Die zu untersuchende Substanz wurde in eine Vena femoralis injiziert. Als Lösungsmittel diente Polydiol 200. Die Substanz wurde an 2 Katzen geprüft, Dosis 0,3 mg/kg i.v..

Die nachfolgende Tabelle enthält die Mittelwerte:

| Substanz | Dosis mg/kg i.v. | diastolische Blutdruck änderung in mmHg | Zunahme von dp/dt in % | Wirkungsdauer (Halbwerts-zeit) |
|---|---|---|---|---|
| A | 0,3 | 0 | + 43 | 18 Minuten |
| B | 0,3 | -21 | + 52 | 16 Minuten |
| C | 0,3 | -11 | + 31 | 11 Minuten |
| D | 0,3 | -37 | + 41 | 4 Minuten |
| E | 0,3 | -16 | + 31 | 7 Minuten |
| F | 0,3 | -24 | + 28 | 10 Minuten |

In diesem Zusammenhang sei darauf hingewiesen, daß bei den biologischen Untersuchungen keine toxischen Nebenwirkungen der Substanzen beobachtet wurden.

Aufgrund ihrer pharmakologischen Eigenschaften eignen sich die erfindungsgemäß hergestellten Verbindungen der allgemeinen Formel I und deren optisch aktive Antipoden sowie deren physiologisch verträgliche Säureadditionssalze mit anorganischen oder organischen Säuren zur Behandlung der chronischen und akuten Herzinsuffizienz unterschiedlicher Genese und/oder zur Prophylaxe arterieller Thromboembolien und arterieller Verschlußkrankheiten.

Hierzu lassen sich die neuen Verbindungen, gegebenenfalls in Kombination mit anderen Wirksubstanzen, in die üblichen pharmazeutischen Anwendungsformen wie Tabletten, Dragées, Pulver, Suspensionen, Suppositorien oder Ampullen einarbei ten. Die Einzeldosis beträgt hierbei beim Erwachsenen 1-4 $^{x}$ täglich bei intravenöser Applikation 1-50 mg, vorzugsweise 2 bis 40 mg, und bei oraler Applikation 5-150 mg, vorzugsweise 5 bis 100 mg.

Die nachfolgenden Beispiele sollen die Erfindung näher erläutern:

Beispiel A

4,4-Dimethyl-4H-3,1-benzoxazin-2-on

Zu einer aus 170 g (7 Mol) Magnesiumspänen, 993 g (7 Mol) Methyljodid in 3,5 l Äther hergestellten Methylmagnesiumjodid-Lösung tropft man bei 15-25°C eine Lösung von 226 g (1,5 Mol) Anthranilsäuremethylester in 1 l Äther zu. Nach 1,5-stündigem Nachrühren wird die Reaktionsmischung auf Ammoniumchlorid-haltiges Eiswasser gegossen, die Ätherphase abgetrennt, die wässrige Phase nochmals mit Äther extrahiert und die vereinigten Ätherphasen mit Ammoniumchlorid-Lösung gewaschen, mit Natriumsulfat getrocknet und der Äther abdestilliert. Das so erhaltene o-Amino-phenyl-dimethyl-carbinol wird ohne Reinigung weiter umgesetzt: Es wird in 1,5 l Chloroform, dem 5 ml Wasser zugefügt sind, gelöst und mit 300 g (2,2 Mol) Kaliumcarbonat versetzt. Dazu tropft man vorsichtig im Verlauf von 2 Stunden 800 ml (1,6 Mol) einer 20%igen Lösung von Phosgen in Toluol, wobei die Temperatur auf 60°C ansteigt. Nach Stehenlassen über Nacht wird 1 l Wasser zugegeben, mit Chloroform extrahiert, die Chloroform-Phase gewaschen und getrocknet und das Lösungsmittel abdestilliert. Der Rückstand wird aus 1,7 l Cyclohexan umkristallisiert.
Ausbeute: 234,2 g (88,4 % der Theorie),
Schmelzpunkt: 107-108°C

Analog wurden hergestellt:

4,4-Diäthyl-4H-3,1-benzoxazin-2-on aus Anthranilsäuremethylester, Äthylmagnesiumbromid und Phosgen.

4H-3,1-Benzoxazin-2-on aus Anthranilsäuremethylester, Lithiumaluminiumhydrid und Phosgen.

Beispiel B

4,4-Dimethyl-6-nitro-4H-3,1-benzoxazin-2-on

Zu 793 ml rauchender Salpetersäure (d = 1,51) werden bei -40°C unter Rühren während 3/4 Stunden 234,1 g (1,32 Mol) 4,4-Dimethyl-4H-3,1-benzoxazin-2-on portionsweise zugegeben und weitere 3/4 Stunden bei -40°C nachgerührt. Die Reaktionsmischung wird dann auf 3 1/2 l Eiswasser gegossen, der Niederschlag abgesaugt, mit Wasser gewaschen, getrocknet und aus Butanol/Dimethylformamid umkristallisiert.
Ausbeute: 251 g (85,6 % der Theorie),
Schmelzpunkt: 244-246°C

Analog wurden hergestellt:

4,4-Diäthyl-6-nitro-4H-3,1-benzoxazin-2-on

6-Nitro-4H-3,1-benzoxazin-2-on

Beispiel C

6-Amino-4.4-dimethyl-4H-3.1-benzoxazin-2-on

251 g (1.13 Mol) 4,4-Dimethyl-6-nitro-4H-3,1-benzoxazin-2-on werden in 1250 ml Dimethylformamid gelöst. mit 30 g Raney-Nickel versetzt und bei 60°C im Autoklaven zunächst 3 Stunden bei 15 bar und dann nochmals 6 Stunden bei 60 bar Wasserstoffdruck hydriert. Man saugt dann vom Katalysator ab, destilliert das Lösungsmittel ab und kristallisiert den Rückstand aus Isopropanol/Diisopropyläther um.
Ausbeute: 205,9 g (94,8 % der Theorie),
Schmelzpunkt: 171-172°C

Analog wurden hergestellt:

6-Amino-4,4-diäthyl-4H-3,1-benzoxazin-2-on

6-Amino-4H-3.1-benzoxazin-2-on

Beispiel D

6-Acetamido-4.4-dimethyl-4H-3.1-benzoxazin-2-on

122.2 g (0,636 Mol) 6-Amino-4,4-dimethyl-4H-3,1-benzoxazin-2-on werden in 800 ml Eisessig gelöst und mit 120 ml Acetanhydrid versetzt. Dabei steigt die Temperatur auf 35°C an. Es wird noch 1/2 Stunde nachgerührt, mit 1 l Äther versetzt und der Niederschlag abgesaugt und mit Äther gewaschen.
Ausbeute: 141,3 g (94,9 % der Theorie),
Schmelzpunkt: 222-223°C

Analog wurden hergestellt:

6-Acetamido-4,4-diäthyl-4H-3,1-benzoxazin-2-on

6-Acetamido-4H-3,1-benzoxazin-2-on

Beispiel E

6-Amino-4.4-dimethyl-7-nitro-4H-3,1-benzoxazin-2-on

Zu 360 ml rauchender Salpetersäure (d = 1,53) werden bei -40°C innerhalb 3/4 Stunden 141,3 g (0,6 Mol) 6-Acetamido-4,4-dimethyl-4H-3,1-benzoxazin-2-on unter Rühren portionsweise zugegeben und noch 1,5 Stunden bei -35°C nachgerührt. Die Re aktionsmischung wird auf Eis gegossen, der Niederschlag abgesaugt, mit Wasser gewaschen und zum Zwecke der Abspaltung der Acetylgruppe 3 Stunden lang in einer Mischung von 500 ml Isopropanol, 500 ml Wasser und 250 ml konzentrierte Salzsäure zum Rückfluß erhitzt. Nach dem Abkühlen wird mit Eiswasser versetzt, der Niederschlag abgesaugt, mit Wasser gewaschen und getrocknet.
Ausbeute: 112 g (78,3 % der Theorie),
Schmelzpunkt: ab 257°C (Zers.)

Analog wurden hergestellt:

6-Amino-4,4-diäthyl-7-nitro-4H-3,1-benzoxazin-2-on

6-Amino-7-nitro-4H-3,1-benzoxazin-2-on

Beispiel F

4,4-Dimethyl-6-isonicotinoylamido-7-nitro-4H-3,1-benzoxazin-2-on

Eine Lösung von 4,75 g (0,02 Mol) 6-Amino-4,4-dimethyl-7-nitro-4H-3,1-benzoxazin-2-on und 4,75 g (0,02 Mol) Isonicotinsäurechlorid in 50 ml Pyridin wird 1 Stunde bei 50°C gerührt. Danach wird Eiswasser zugegeben, der Niederschlag abgesaugt, mit Wasser gewaschen und aus Butanol/Dimethylformamid umkristallisiert.
Ausbeute: 5,94 g (86,7 % der Theorie),
Schmelzpunkt: 265-267°C

Analog wurden hergestellt:

6-Isonicotinoylamido-7-nitro-1,4,4-trimethyl-4H-3,1-benzoxazin-2-on
Schmelzpunkt: 232-233°C

6-Nicotinoylamido-7-nitro-1,4,4-trimethyl-4H-3,1-benzoxazin-2-on
Schmelzpunkt: 224-226°C

4,4-Dimethyl-6-nicotinoylamido-7-nitro-4H-3,1-benzoxazin-2-on
Schmelzpunkt: 277-279°C

6-(4-Methoxy-benzoylamido)-7-nitro-1,4,4-trimethyl-4H-3,1-benzoxazin-2-on
Schmelzpunkt: 193-195°C

4,4-Dimethyl-6-(4-methoxy-benzoylamido)-7-nitro-4H-3,1-benzoxazin-2-on
Schmelzpunkt: > 300°C

1-Äthyl-4,4-dimethyl-6-isonicotinoylamido-7-nitro-4H-3,1-benzoxazin-2-on
Schmelzpunkt: 196-197°C

4,4-Dimethyl-6-isonicotinoylamido-1-(2-methoxy-äthyl)-7-nitro-4H-3,1-benzoxazin-2-on
Schmelzpunkt: 186-188°C

6-Benzoylamido-4,4-dimethyl-7-nitro-4H-3,1-benzoxazin-2-on
Schmelzpunkt: ab 287°C (Zers.)

4,4-Dimethyl-1-(2-methoxy-äthyl)-6-nicotinoylamido-7-nitro-4H-3,1-benzoxazin-2-on
Schmelzpunkt: 140°C (Zers.)

4,4-Dimethyl-1-isopropyl-6-isonicotinoylamido-7-nitro-4H-3,1-benzoxazin-2-on
Schmelzpunkt: 254-256°C (Zers.)

4,4-Dimethyl-1-isopropyl-6-nicotinoylamido-7-nitro-4H-3,1-benzoxazin-2-on
Schmelzpunkt: 177-178°C

1-Benzyl-4,4-dimethyl-6-isonicotinoylamido-7-nitro-4H-3,1-benzoxazin-2-on
Schmelzpunkt: 181-182°C

1-Benzyl-4,4-dimethyl-6-nicotinoylamido-7-nitro-4H-3,1-benzoxazin-2-on
Schmelzpunkt: 186-187°C

1-Benzyl-4,4-dimethyl-6-(4-methoxy-benzoylamido)-7-nitro-4H-3,1-benzoxazin-2-on
Schmelzpunkt: 156-157°C

4,4-Dimethyl-6-isonicotinoylamido-1-n-propyl-7-nitro-4H-3,1-benzoxazin-2-on
Schmelzpunkt: 221-222°C

4,4-Dimethyl-6-(4-methoxy-benzoylamido)-7-nitro-1-n-propyl-4H-3,1-benzoxazin-2-on
Schmelzpunkt: 201-202°C

4.4-Dimethyl-6-nicotinoylamido-7-nitro-1-n-propyl-4H-3.1-benzoxazin-2-on
Schmelzpunkt: 164-165°C

1-n-Butyl-4.4-dimethyl-6-(4-methoxy-benzoylamido)-7-nitro-4H-3,1-benzoxazin-2-on
Schmelzpunkt: 174-175°C

1-n-Butyl-4,4-dimethyl-6-isonicotinoylamido-7-nitro-4H-3,1-benzoxazin-2-on
Schmelzpunkt: 171-172°C

1-n-Butyl-4,4-dimethyl-6-nicotinoylamido-7-nitro-4H-3,1-benzoxazin-2-on
Schmelzpunkt: 152-153°C

1-Äthyl-4,4-dimethyl-6-(4-methoxy-benzoylamido)-7-nitro-4H-3,1-benzoxazin-2-on
Schmelzpunkt: 176-178°C

1-Äthyl-4,4-dimethyl-6-nicotinoylamido-7-nitro-4H-3,1-benzoxazin-2-on
Schmelzpunkt: 188-189°C

4.4-Dimethyl-6-(3.5-di-tert.butyl-4-hydroxy-benzoylamido)-7-nitro-4H-3.1-benzoxazin-2-on
Schmelzpunkt: > 290°C

1-Äthyl-4.4-dimethyl-6-(3,5-di-tert.butyl-4-hydroxy-benzoylamido)-7-nitro-4H-3,1-benzoxazin-2-on
Schmelzpunkt: 234-235°C

6-(3.5-Di-tert.butyl-4-hydroxy-benzoylamido)-7-nitro-1,4,4-trimethyl-4H-3,1-benzoxazin-2-on
Schmelzpunkt: 247-248°C

6-Isonicotinoylamido-7-nitro-4H-3,1-benzoxazin-2-on
Schmelzpunkt: 290°C (Zers.)

6-Benzoylamido-7-nitro-4H-3,1-benzoxazin-2-on
Schmelzpunkt: Öl

6-Acetamido-7-nitro-4H-3,1-benzoxazin-2-on
Schmelzpunkt: ab 250°C (Zers.)

6-Isonicotinoylamido-1-methyl-7-nitro-4H-3,1-benzoxazin-2-on
Schmelzpunkt: 208-209°C

6-Nicotinoylamido-1-methyl-7-nitro-4H-3,1-benzoxazin-2-on
Schmelzpunkt: 230-231°C

1-Methyl-6-(4-methoxy-benzoylamido)-7-nitro-4H-3,1-benzoxazin-2-on
Schmelzpunkt: 207-208°C

4,4-Diäthyl-6-(4-methoxy-benzoylamido)-7-nitro-4H-3,1-benzoxazin-2-on
Schmelzpunkt: 205-207°C

4,4-Diäthyl-6-isonicotinoylamido-7-nitro-4H-3,1-benzoxazin-2-on
Schmelzpunkt: 197-199°C

4,4-Diäthyl-6-nicotinoyl-amido-7-nitro-4H-3,1-benzoxazin-2-on
Schmelzpunkt: 108-110°C

4,4-Diäthyl-1-methyl-6-isonicotinoylamido-7-nitro-4H-3,1-benzoxazin-2-on
Schmelzpunkt: 190-192°C

4,4-Diäthyl-6-(4-methoxy-benzoylamido)-1-methyl-7-nitro-4H-3,1-benzoxazin-2-on
Schmelzpunkt: 176-177°C

4,4-Diäthyl-1-methyl-6-nicotinoylamido-7-nitro-4H-3,1-benzoxazin-2-on
Schmelzpunkt: 143-144°C

6-Isonicotinoylamido-7-nitro-1,4,4-triäthyl-4H-3,1-benzoxazin-2-on
Schmelzpunkt: 179-180°C

6-Nicotinoylamido-7-nitro-1,4,4-triäthyl-4H-3,1-benzoxazin-2-on
Schmelzpunkt: 162-163°C

6-(4-Methoxy-benzoylamido)-7-nitro-1,4,4-triäthyl-4H-3,1-benzoxazin-2-on
Schmelzpunkt: 144-145°C

4,4-Dimethyl-6-(2-phenyl-propionylamido)-7-nitro-4H-3,1-benzoxazin-2-on
Schmelzpunkt: 220-222°C

6-(2,4-Dimethoxy-benzoylamido)-4,4-dimethyl-7-nitro-4H-3,1-benzoxazin-2-on
Schmelzpunkt: 257-259°C

4,4-Dimethyl-7-nitro-6-phenylacetamido-4H-3,1-benzoxazin-2-on.
Schmelzpunkt:240-242°C

6-Butyrylamido-4,4-dimethyl-7-nitro-4H-3,1-benzoxazin-2-on
Schmelzpunkt: 281°C

4,4-Dimethyl-6-(2-methyl-valerylamido)-7-nitro-4H-3,1-benzoxazin-2-on
Schmelzpunkt: 184-185°C

4,4-Dimethyl-6-(4-methylsulfonyl-benzoylamido)-7-nitro-4H-3,1-benzoxazin-2-on
Schmelzpunkt: ab 270°C (Zers.)

6-(4-Amino-3,5-dibrom-benzoylamido)-4,4-dimethyl-7-nitro-4H-3,1-benzoxazin-2-on
Schmelzpunkt: 286-288°C

1-Äthyl-6-(4-amino-3,5-dibrom-benzoylamido)-4,4-dimethyl-7-nitro-4H-3,1-benzoxazin-2-on
Schmelzpunkt: 216-218°C

6-(4-Amino-3,5-dichlor-benzoylamido)-4,4-dimethyl-7-nitro-4H-3,1-benzoxazin-2-on
Schmelzpunkt: ab 288°C

1-Äthyl-6-(4-amino-3,5-dichlor-benzoylamido)-4,4-dimethyl-7-nitro-4H-3,1-benzoxazin-2-on
Schmelzpunkt: 246-247°C

6-(4-Chinolin-carbonamido)-4,4-dimethyl-7-nitro-4H-3,1-benzoxazin-2-on
Schmelzpunkt: 269-270°C

1-Äthyl-4,4-dimethyl-6-(2-thenoylamido)-7-nitro-4H-3,1-benzoxazin-2-on
Schmelzpunkt: 177-179°C

1-Äthyl-4,4-dimethyl-6-(2-furoylamido)-7-nitro-4H-3,1-benzoxazin-2-on
Schmelzpunkt: 215-217°C

4,4-Dimethyl-6-(2-furoylamido)-7-nitro-4H-3,1-benzoxazin-2-on
Schmelzpunkt: 263-265°C

4,4-Dimethyl-7-nitro-6-(2-thenoylamido)-4H-3,1-benzoxazin-2-on

Schmelzpunkt: ab 227°C

4.4-Dimethyl-6-(4-methylmercapto-benzoylamido)-7-nitro-4H-3.1-benzoxazin-2-on
Schmelzpunkt: 292-294°C

4.4-Dimethyl-7-nitro-6-[3-(4-pyridyl)-propionylamido]-4H-3,1-benzoxazin-2-on
Schmelzpunkt: 257-258°C

4,4-Dimethyl-6-(2-morpholino-nicotinoylamido)-7-nitro-4H-3,1-benzoxazin-2-on
Schmelzpunkt: 257-259°C

4,4-Dimethyl-7-nitro-6-(2-pyrazinoylamido)-4H-3,1-benzoxazin-2-on
Schmelzpunkt: > 300°C

4,4-Dimethyl-1-isopropyl-6-(4-methoxy-benzoylamido)-7-nitro-4H-3,1-benzoxazin-2-on
Schmelzpunkt: 179-181°C

6-(4-Cyan-benzoylamido)-4,4-dimethyl-7-nitro-4H-3,1-benzoxazin-2-on
Schmelzpunkt: 276-278°C

4.4-Dimethyl-6-(3-furoylamido)-7-nitro-4H-3,1-benzoxazin-2-on
Schmelzpunkt: 252-253°C

1-Äthyl-4,4-dimethyl-6-(3-furoylamido)-7-nitro-4H-3,1-benzoxazin-2-on
Schmelzpunkt: 206-208°C

4,4-Dimethyl-7-nitro-6-(3-thenoylamido)-4H-3,1-benzoxazin-2-on
Schmelzpunkt: 285-287°C

1-Äthyl-4,4-dimethyl-7-nitro-6-(3-thenoylamido)-4H-3,1-benzoxazin-2-on
Schmelzpunkt: 277-278°C

1-Äthyl-4,4-dimethyl-7-nitro-6-(2-pyrazinoylamido)-4H-3,1-benzoxazin-2-on
Schmelzpunkt: 221°C

4.4-Dimethyl-1-isopropyl-7-nitro-6-(2-pyrazinoylamido)-4H-3,1-benzoxazin-2-on
Schmelzpunkt: 253°C

7-Nitro-6-(2-pyrazinoylamido)-1.4,4-trimethyl-4H-3,1-benzoxazin-2-on
Schmelzpunkt: 295-296°C

4,4-Dimethyl-7-nitro-1-n-propyl-6-(2-pyrazinoylamido)-4H-3,1-benzoxazin-2-on
Schmelzpunkt: 253°C

1-n-Butyl-4,4-dimethyl-7-nitro-6-(2-pyrazinoylamido)-4H-3,1-benzoxazin-2-on
Schmelzpunkt: 163-164°C

4,4-Dimethyl-1-(2-methoxy-äthyl)-7-nitro-6-(2-pyrazinoylamido)-4H-3,1-benzoxazin-2-on
Schmelzpunkt: 162-163°C.

Beispiel G

6,7-Diamino-4,4-dimethyl-4H-3,1-benzoxazin-2-on-dihydrochlorid

35,6 g (0,15 Mol) 6-Amino-4,4-dimethyl-7-nitro-4H-3,1-benzoxazin-2-on werden in 500 ml Methanol und 60 ml 7,5 n methanolischer Salzsäure gelöst, mit 3,5 g 10%iger Palladium/Kohle versetzt und im Autoklaven bei Raumtemperatur und 5 bar Wasserstoffdruck während 45 Minuten hydriert. Anschließend wird vom Katalysator abgesaugt und das Filtrat mit Äther auf 2 l verdünnt. Der Niederschlag wird abgesaugt, mit Äther gewaschen und getrocknet.
Ausbeute: 42 g (99,9 % der Theorie),
Schmelzpunkt: 218-220°C

Beispiel 1

8,8-Dimethyl-2-(4-pyridyl)-8-hydro-(5H)-imidazo[5,4-g][3,1]-benzoxazin-6-on

5,8 g (16,9 mMol) 4,4-Dimethyl-6-isonicotinoylamido-7-nitro-4H-3,1-benzoxazin-2-on werden in 100 ml Eisessig gelöst, mit 1 g Raney-Nickel versetzt und während 1 Stunde bei 80°C und 5 bar Wasserstoffdruck im Autoklaven hydriert. Das ausgefallene Reaktionsprodukt wird durch Zugabe von Dimethylformamid und Methanol gelöst und in der Hitze vom Katalysator abfiltriert. Das Filtrat wird zur Trockene eingeengt, mit Wasser aufgerührt, durch Zugabe von konz. wäßrigem Ammoniak alkalisch gestellt und abgesaugt.
Ausbeute: 2,2 g (44,5 % der Theorie),
Schmelzpunkt: > 300°C (aus Isopropanol/Essigester)
$C_{16}H_{14}N_4O_2$ (294,31)
Ber.: C 65,30 H 4,79 N 19,04
Gef.: C 65,03 H 4,79 N 18,84

Beispiel 2

2-(4-Pyridyl)-5,8,8-trimethyl-8-hydro-(5H)-imidazo[5,4-g][3,1]benzoxazin-6-on $\times$ 4 $H_2O$

Hergestellt analog Beispiel 1 aus 6-Isonicotinoylamido-7-nitro-1,4,4-trimethyl-4H-3,1-benzoxazin-2-on.
Ausbeute: 77,6 % der Theorie (Isopropanol),
Schmelzpunkt: 137-139°C
$C_{17}H_{16}N_4O_2 \times 4\ H_2O$ (380,40)
Ber.: C 53,96 H 5,86 N 14,81
Gef.: C 53,75 H 6,04 N 14,66

Beispiel 3

2-(3-Pyridyl)-5,8,8-trimethyl-8-hydro-(5H)-imidazo[5,4-g][3,1]benzoxazin-6-on $\times$ 0,5 $H_2O$

Hergestellt analog Beispiel 1 aus 6-Nicotinoylamido-7-nitro-1,4,4-trimethyl-4H-3,1-benzoxazin-2-on.
Ausbeute: 69,5 % der Theorie,
Schmelzpunkt: 285°C (Isopropanol)
$C_{17}H_{16}N_4O_2 \times 0{,}5\ H_2O$ (317,35)
Ber.: C 64,32 H 5,40 N 17,65
Gef.: C 64,52 H 5,46 N 17,61

Beispiel 4

8.8-Dimethyl-2-(3-pyridyl)-8-hydro-(5H)-imidazo[5.4-g][3.1]benzoxazin-6-on

Hergestellt analog Beispiel 1 aus 4,4-Dimethyl-6-nicotinoylamido-7-nitro-4H-3,1-benzoxazin-2-on.
Ausbeute: 48.3 % der Theorie.
Schmelzpunkt: 293°C (Dioxan/Wasser)
$C_{15}H_{14}N_4O_2$ (294,32)
Ber.: C 65,30 H 4,79 N 18,57
Gef.: C 65,12 H 4,96 N 18,47

Beispiel 5

2-(4-Methoxy-phenyl)-5.8.8-trimethyl-8-hydro-(5H)-imidazo[5,4-g][3,1]benzoxazin-6-on

Hergestellt analog Beispiel 1 aus 6-(4-Methoxy-benzoylamido)-7-nitro-1,4,4-trimethyl-4H-3.1-benzoxazin-2-on.
Ausbeute: 70.0 % der Theorie.
Schmelzpunkt: 276-277°C (Isopropanol/Dioxan)
$C_{19}H_{19}N_3O_3$ (337,38)
Ber.: C 67.64 H 5.68 N 12.45
Gef.: C 67,84 H 5,47 N 12,47

Beispiel 6

8.8-Dimethyl-2-(4-methoxy-phenyl)-8-hydro-(5H)-imidazo[5,4-g][3,1]benzoxazin-6-on

Hergestellt analog Beispiel 1 aus 4.4-Dimethyl-6-(4-methoxybenzoylamido)-7-nitro-4H-3,1-benzoxazin-2-on.
Ausbeute: 76,9 % der Theorie.
Schmelzpunkt: 278°C (Dimethylformamid/Wasser)
$C_{18}H_{17}N_3O_3$ (323,35)
Ber.: C 66,86 H 5,30 N 13,00
Gef.: C 66,70 H 5,47 N 12,89

Beispiel 7

5-Äthyl-8,8-dimethyl-2-(4-pyridyl)-8-hydro-(5H)-imidazo[5,4-g][3,1]benzoxazin-6-on

Hergestellt analog Beispiel 1 aus 1-Äthyl-4,4-dimethyl-6-isonicotinoylamido-7-nitro-4H-3.1-benzoxazin-2-on.
Ausbeute: 52,0 % der Theorie.
Schmelzpunkt: 281-283°C (Essigester/Isopropanol)
$C_{18}H_{18}N_4O_2$ (322,37)
Ber.: C 67,07 H 5,63 N 17,38
Gef.: C 67,00 H 5,94 N 17,32

Beispiel 8

8,8-Dimethyl-5-(2-methoxy-äthyl)-2-(4-pyridyl)-8-hydro-(5H)-imidazo[5,4-g][3,1]benzoxazin-6-on

Hergestellt analog Beispiel 1 aus 4,4-Dimethyl-6-isonicotinoylamido-1-(2-methoxy-äthyl)-7-nitro-4H-3,1-benzoxazin-2-on.
Ausbeute: 57,3 % der Theorie.
Schmelzpunkt: 140°C (Essigester)
$C_{19}H_{20}N_4O_3$ (352,40)
Ber.: C 64,76 H 5,72 N 15,90
Gef.: C 64,90 H 5,78 N 16,05

Beispiel 9

8,8-Dimethyl-2-phenyl-8-hydro-(5H)-imidazo[5,4-g][3,1]benzoxazin-6-on

Hergestellt analog Beispiel 1 aus 6-Benzoylamido-4,4-dimethyl-7-nitro-4H-3,1-benzoxazin-2-on.
Ausbeute: 33 % der Theorie,
Schmelzpunkt: 238-239°C (Isopropanol)
$C_{17}H_{15}N_3O_2$ (293,33)
Ber.: C 69,61 H 5,15 N 14,33
Gef.: C 69,77 H 5,31 N 14,13

Beispiel 10

8,8-Dimethyl-5-(2-methoxy-äthyl)-2-(3-pyridyl)-8-hydro-(5H)-imidazo[5,4-g][3,1]benzoxazin-6-on $\times$ 0,9 $H_2O$

Hergestellt analog Beispiel 1 aus 4,4-Dimethyl-1-(2-methoxy-äthyl)-6-nicotinoylamido-7-nitro-4H-3,1-benzoxazin-2-on.
Ausbeute: 44 % der Theorie,
Schmelzpunkt: 111°C (Essigester/Isopropanol)
$C_{19}H_{20}N_4O_3 \times 0,9\ H_2O$ (368,61)
Ber.: C 61,91 H 5,96 N 15,20
Gef.: C 61,99 H 5,92 N 15,49

Beispiel 11

8,8-Dimethyl-5-isopropyl-2-(4-pyridyl)-8-hydro-(5H)-imidazo[5,4-g][3,1]benzoxazin-6-on

Hergestellt analog Beispiel 1 aus 4,4-Dimethyl-1-isopropyl-6-isonicotinoylamido-7-nitro-4H-3,1-benzoxazin-2-on.
Ausbeute: 68 % der Theorie,
Schmelzpunkt: 165°C (Essigester)
$C_{19}H_{20}N_4O_2$ (336,40)
Ber.: C 67,84 H 5,99 N 16,66
Gef.: C 67,60 H 5,83 N 16,63

Beispiel 12

8,8-Dimethyl-5-isopropyl-2-(3-pyridyl)-8-hydro-(5H)-imidazo[5,4-g][3.1]benzoxazin-6-on

Hergestellt analog Beispiel 1 aus 4,4-Dimethyl-1-isopropyl-6-isonicotinoylamido-7-nitro-4H-3,1-benzoxazin-2-on.
Ausbeute: 35.6 % der Theorie,
Schmelzpunkt: 269-271 °C (Essigester/Isopropanol)
$C_{19}H_{20}N_4O_2$ (336,40)
Ber.: C 67,84 H 6.10 N 16,64
Gef.: C 68,05 H 5,94 N 16,60

Beispiel 13

5-Benzyl-8,8-dimethyl-2-(4-pyridyl)-8-hydro-(5H)-imidazo[5,4-g][3.1]benzoxazin-6-on

Hergestellt analog Beispiel 1 aus 1-Benzyl-4,4-dimethyl-6-isonicotinoylamido-7-nitro-4H-3,1-benzoxazin-2-on.
Ausbeute: 54,2 % der Theorie,
Schmelzpunkt: 227-228 °C (Essigester)
$C_{22}H_{20}N_4O_2$ (384.44)
Ber.: C 71,86 H 5,24 N 14,57
Gef.: C 71,66 H 5,34 N 14,31

Beispiel 14

5-Benzyl-8,8-dimethyl-2-(3-pyridyl)-8-hydro-(5H)-imidazo[5,4-g][3,1]benzoxazin-6-on

Hergestellt analog Beispiel 1 aus 1-Benzyl-4,4-dimethyl-6-nicotinoylamido-7-nitro-4H-3,1-benzoxazin-2-on.
Ausbeute: 36 % der Theorie,
Schmelzpunkt: 258-259 °C (Essigester)
$C_{22}H_{20}N_4O_2$ (384,44)
Ber.: C 71,86 H 5,24 N 14,57
Gef.: C 71,83 H 5,32 N 14,39

Beispiel 15

5-Benzyl-8,8-dimethyl-2-(4-methoxy-phenyl)-8-hydro-(5H)-imidazo[5.4-g][3,1]benzoxazin-6-on

Hergestellt analog Beispiel 1 aus 1-Benzyl-4,4-dimethyl-6-(4-methoxy-benzoylamido)-7-nitro-4H-3,1-benzoxazin-2-on.
Ausbeute: 57,4 % der Theorie,
Schmelzpunkt: 239-241 °C (Essigester)
$C_{25}H_{23}N_3O_3$ (413,48)
Ber.: C 72,62 H 5,61 N 10,16
Gef.: C 72,80 H 5,71 N 10,00

Beispiel 16

8,8-Dimethyl-5-n-propyl-2-(4-pyridyl)-8-hydro-(5H)-imidazo[5,4-g][3.1]benzoxazin-6-on

Hergestellt analog Beispiel 1 aus 4,4-Dimethyl-6-isonicotinoylamido-1-n-propyl-7-nitro-4H-3.1-benzoxazin-2-on.
Ausbeute: 76 % der Theorie,
Schmelzpunkt: 229-231 °C (Essigester)
$C_{19}H_{20}N_4O_2$ (336,40)
Ber.: C 67,84 H 5,99 N 16,66
Gef.: C 67,76 H 6,12 N 16,55

Beispiel 17

8,8-Dimethyl-2-(4-methoxy-phenyl)-5-n-propyl-8-hydro-(5H)-imidazo[5,4-g][3,1]benzoxazin-6-on

Hergestellt analog Beispiel 1 aus 4,4-Dimethyl-6-(4-methoxybenzoylamido)-7-nitro-1-n-propyl-4H-3.1-benzoxazin-2-on.
Ausbeute: 52 % der Theorie,
Schmelzpunkt: 245-246 °C (Essigester/Isopropanol)
$C_{21}H_{23}N_3O_3$ (365.43)
Ber.: C 69,02 H 6.34 N 11,50
Gef.: C 68,90 H 6.41 N 11,39

Beispiel 18

8,8-Dimethyl-5-n-propyl-2-(3-pyridyl)-8-hydro-(5H)-imidazo[5.4-g][3.1]benzoxazin-6-on

Hergestellt analog Beispiel 1 aus 4,4-Dimethyl-6-nicotinoylamido)-7-nitro-1-n-propyl-4H-3,1-benzoxazin-2-on.
Ausbeute: 30 % der Theorie,
Schmelzpunkt: 199-201 °C (Essigester)
$C_{19}H_{20}N_4O_2$ (336,40)
Ber.: C 67,84 H 5,99 N 16,66
Gef.: C 67,75 H 6,12 N 16,58

Beispiel 19

5-n-Butyl-8,8-dimethyl-2-(4-methoxy-phenyl)-8-hydro-(5H)-imidazo[5,4-g][3.1]benzoxazin-6-on

Hergestellt analog Beispiel 1 aus 1-n-Butyl-4,4-dimethyl-6-(4-methoxy-benzoylamido)-7-nitro-4H-3.1-benzoxazin-2-on.
Ausbeute: 52 % der Theorie,
Schmelzpunkt: 227-229 °C (Isopropanol/Essigester)
$C_{22}H_{25}N_3O_3$ (379,46)
Ber.: C 69,64 H 6,64 N 11,07
Gef.: C 69,41 H 6,72 N 11,04

Beispiel 20

5-n-Butyl-8.8-dimethyl-2-(4-pyridyl)-8-hydro-(5H)-imidazo[5,4-g][3.1]benzoxazin-6-on

Hergestellt analog Beispiel 1 aus 1-n-Butyl-4.4-dimethyl-6-isonicotinoylamido-7-nitro-4H-3,1-benzoxazin-2-on.
Ausbeute: 70 % der Theorie,
Schmelzpunkt: 226-227 °C (Essigester)
$C_{20}H_{22}N_4O_2$ (350,42)
Ber.: C 68,55 H 6,33 N 15,99
Gef.: C 68,35 H 6,50 N 15,70

Beispiel 21

5-n-Butyl-8.8-dimethyl-2-(3-pyridyl)-8-hydro-(5H)-imidazo[5,4-g][3,1]benzoxazin-6-on

Hergestellt analog Beispiel 1 aus 1-n-Butyl-4,4-dimethyl-6-nicotinoylamido-7-nitro-4H-3,1-benzoxazin-2-on.
Ausbeute: 75,8 % der Theorie,
Schmelzpunkt: 220-221 °C (Essigester)
$C_{20}H_{22}N_4O_2$ (350.42)
Ber.: C 68,55 H 6,33 N 15,99
Gef.: C 68.47 H 6.56 N 15.94

Beispiel 22

5-Äthyl-8.8-dimethyl-2-(4-methoxy-phenyl)-8-hydro-(5H)-imidazo[5,4-g][3.1]benzoxazin-6-on

Hergestellt analog Beispiel 1 aus 1-Äthyl-4,4-dimethyl-6-(4-methoxy-benzoylamido)-7-nitro-4H-3,1-benzoxazin-2-on.
Ausbeute: 60 % der Theorie,
Schmelzpunkt: 285-286 °C (Essigester/Isopropanol)
$C_{20}H_{21}N_3O_3$ (351,41)
Ber.: C 68,36 H 6,02 N 11,96
Gef.: C 68,45 H 6,09 N 11,96

Beispiel 23

5-Äthyl-8,8-dimethyl-2-(3-pyridyl)-8-hydro-(5H)-imidazo[5,4-g][3,1]benzoxazin-6-on

Hergestellt analog Beispiel 1 aus 1-Äthyl-4,4-dimethyl-6-nicotinoylamido-7-nitro-4H-3,1-benzoxazin-2-on.
Ausbeute: 53 % der Theorie,
Schmelzpunkt: 274-276 °C (Essigester/Isopropanol)
$C_{18}H_{18}N_4O_2$ (322,37)
Ber.: C 67,07 H 5,63 N 17,38
Gef.: C 67,00 H 5,84 N 17,20

Beispiel 24

8,8-Dimethyl-2-(3,5-di-tert.butyl-4-hydroxy-phenyl)-8-hydro-(5H)-imidazo[5,4-g][3,1]benzoxazin-6-on

Hergestellt analog Beispiel 1 aus 4,4-Dimethyl-6-(3,5-di-tert.butyl-4-hydroxy-benzoylamido)-7-nitro-4H-3,1-benzoxazin-2-on.
Ausbeute: 31 % der Theorie,
Schmelzpunkt: > 300°C (Isopropanol)
$C_{25}H_{31}N_3O_3$ (421,54)
Ber.: C 71,23 H 7,41 N 9,97
Gef.: C 71,34 H 7,43 N 9,84

Beispiel 25

5-Äthyl-2-(3,5-di-tert.butyl-4-hydroxy-phenyl)-8,8-dimethyl-8-hydro-(5H)-imidazo[5,4-g][3,1]benzoxazin-6-on

Hergestellt analog Beispiel 1 aus 1-Äthyl-4,4-dimethyl-6-(3,5-di-tert.butyl-4-hydroxy-benzoylamido)-7-nitro-4H-3,1-benzoxazin-2-on.
Ausbeute: 56 % der Theorie,
Schmelzpunkt: > 300°C (Essigester/Isopropanol)
$C_{27}H_{35}N_3O_3$ (449.59)
Ber.: C 72,13 H 7,85 N 9.35
Gef.: C 72,21 H 7,97 N 9,39

Beispiel 26

2-(3,5-Di-tert.butyl-4-hydroxy-phenyl)-5,8,8-trimethyl-8-hydro-(5H)-imidazo[5,4-g][3,1]benzoxazin-6-on

Hergestellt analog Beispiel 1 aus 6-(3,5-Di-tert.butyl-4-hydroxy-benzoylamido)-7-nitro-1,4,4-trimethyl-4H-3,1-benzoxazin-2-on.
Ausbeute: 63 % der Theorie,
Schmelzpunkt: > 300°C (Essigester)
$C_{26}H_{33}N_3O_3$ (435,57)
Ber.: C 71,70 H 7,64 N 9,65
Gef.: C 71,52 H 7,71 N 9,54

Beispiel 27

2-(4-Pyridyl)-8-hydro-(5H)-imidazo[5,4-g][3,1]benzoxazin-6-on

Hergestellt analog Beispiel 1 aus 6-Isonicotinoylamido-7-nitro-4H-3,1-benzoxazin-2-on.
Ausbeute: 30,1 % der Theorie,
Schmelzpunkt: > 290°C (Dimethylformamid/n-Butanol)
$C_{14}H_{10}N_4O_2$ (226,26)
Ber.: C 63,15 H 3,79 N 21,04
Gef.: C 63,20 H 3,99 N 21,16

Beispiel 28

2-Phenyl-8-hydro-(5H)-imidazo[5,4-g][3.1]benzoxazin-6-on-hydrochlorid

Hergestellt analog Beispiel 1 aus 6-Benzoylamido-7-nitro-4H-3,1-benzoxazin-2-on.
Ausbeute: 11 % der Theorie.
Schmelzpunkt: > 300°C (Äthanol/Wasser)
$C_{15}H_{12}ClN_3O_2$ (301,73)
Ber.: C 59,71 H 4,01 N 13,93 Cl 11,75
Gef.: C 59,79 H 4,29 N 13,93 Cl 11,50

Beispiel 29

2-Methyl-8-hydro-(5H)-imidazo[5,4-g][3,1]benzoxazin-6-on-hydrochlorid

Hergestellt analog Beispiel 1 aus 6-Acetamido-7-nitro-4H-3,1-benzoxazin-2-on.
Ausbeute: 12,5 % der Theorie,
Schmelzpunkt: > 305°C (n-Butanol/Wasser)
$C_{10}H_{10}ClN_3O_2$ (239,67)
Ber.: C 50.12 H 4,21 N 17,53 Cl 14.79
Gef.: C 49.96 H 4.30 N 17.64 Cl 14.75

Beispiel 30

5-Methyl-2-(4-pyridyl)-8-hydro-(5H)-imidazo[5,4-g][3.1]benzoxazin-6-on x $H_2O$

Hergestellt analog Beispiel 1 aus 6-Isonicotinoylamido-1-methyl-7-nitro-4H-3,1-benzoxazin-2-on.
Ausbeute: 44 % der Theorie,
Schmelzpunkt: 290°C (Zers., Dioxan)
$C_{15}H_{12}N_4O_2$ x $H_2O$ (298,30)
Ber.: C 60,40 H 4,73 N 18,78
Gef.: C 60,10 H 4,69 N 18,70

Beispiel 31

5-Methyl-2-(3-pyridyl)-8-hydro-(5H)-imidazo[5,4-g][3.1]benzoxazin-6-on x 0,5 $H_2O$

Hergestellt analog Beispiel 1 aus 6-Nicotinoylamido-1-methyl-7-nitro-4H-3,1-benzoxazin-2-on.
Ausbeute: 26 % der Theorie,
Schmelzpunkt: 264-266°C (Dioxan)
$C_{15}H_{12}N_4O_2$ x 0,5 $H_2O$ (289,29)
Ber.: C 62,28 H 4,53 N 19,37
Gef.: C 62,18 H 4,55 N 19,48

Beispiel 32

2-(4-Methoxy-phenyl)-5-methyl-8-hydro-(5H)-imidazo[5,4-g][3,1]benzoxazin-6-on x 0,5 $H_2O$

Hergestellt analog Beispiel 1 aus 1-Methyl-6-(4-methoxybenzoylamido)-7-nitro-4H-3,1-benzoxazin-2-on.
Ausbeute: 51 % der Theorie,
Schmelzpunkt: ab 258°C (Essigester/Isopropanol)
$C_{15}H_{12}N_4O_2$ x 0,5 $H_2O$ (289,29)
Ber.: C 64,14 H 5,07 N 13,20
Gef.: C 64,25 H 5,08 N 13,20

Beispiel 33

8,8-Diäthyl-2-(4-methoxy-phenyl)-8-hydro-(5H)-imidazo[5,4-g][3,1]benzoxazin-6-on $\times$ $H_2O$

Hergestellt analog Beispiel 1 aus 4,4-Diäthyl-6-(4-methoxybenzoylamido)-7-nitro-4H-3,1-benzoxazin-2-on.
Ausbeute: 50,1 % der Theorie,
Schmelzpunkt: 186-188°C (Essigester)
$C_{20}H_{21}N_3O_3 \times H_2O$ (369,42)
Ber.: C 65,03 H 6,28 N 11,37
Gef.: C 65,16 H 6,34 N 11,32

Beispiel 34

8,8-Diäthyl-2-(4-pyridyl)-8-hydro-(5H)-imidazo[5,4-g][3,1]benzoxazin-6-on

Hergestellt analog Beispiel 1 aus 4,4-Diäthyl-6-isonicotino ylamido-7-nitro-4H-3,1-benzoxazin-2-on.
Ausbeute: 50,6 % der Theorie,
Schmelzpunkt: 284°C (Zers., Essigester)
$C_{18}H_{18}N_4O_2$ (322,37)
Ber.: C 67,07 H 5,63 N 17,38
Gef.: C 66,87 H 5,72 N 17,20

Beispiel 35

8,8-Diäthyl-2-(3-pyridyl)-8-hydro-(5H)-imidazo[5,4-g][3,1]benzoxazin-6-on

Hergestellt analog Beispiel 1 aus 4,4-Diäthyl-6-nicotinoylamido-7-nitro-4H-3,1-benzoxazin-2-on.
Ausbeute: 63,6 % der Theorie,
Schmelzpunkt: ab 302°C (Essigester)
$C_{18}H_{18}N_4O_2$ (322,37)
Ber.: C 67,07 H 5,63 N 17,38.
Gef.: C 66,94 H 5,75 N 17,24

Beispiel 36

8,8-Diäthyl-5-methyl-2-(4-pyridyl)-8-hydro-(5H)-imidazo[5,4-g][3,1]benzoxazin-6-on

Hergestellt analog Beispiel 1 aus 4,4-Diäthyl-1-methyl-6-isonicotinoylamido-7-nitro-4H-3,1-benzoxazin-2-on.
Ausbeute: 62 % der Theorie,
Schmelzpunkt: 194-195°C (Essigester)
$C_{19}H_{20}N_4O_2$ (336,40)
Ber.: C 67,84 H 5,99 N 16,66
Gef.: C 67,83 H 6,04 N 16,80

Beispiel 37

8,8-Diäthyl-2-(4-methoxy-phenyl)-5-methyl-8-hydro-(5H)-imidazo[5,4-g][3,1]benzoxazin-6-on

Hergestellt analog Beispiel 1 aus 4,4-Diäthyl-6-(4-methoxybenzoylamido)-1-methyl-7-nitro-4H-3.1-benzoxazin-2-on.
Ausbeute: 64 % der Theorie.
Schmelzpunkt: 200-201 °C (Essigester)
$C_{21}H_{23}N_3O_3$ (365,43)
Ber.: C 69,02 H 6,34 N 11,50
Gef.: C 69,19 H 6,48 N 11,60

Beispiel 38

8,8-Diäthyl-5-methyl-2-(3-pyridyl)-8-hydro-(5H)-imidazo[5.4-g][3,1]benzoxazin-6-on

Hergestellt analog Beispiel 1 aus 4,4-Diäthyl-1-methyl-6-nicotinoylamido-7-nitro-4H-3,1-benzoxazin-2-on.
Ausbeute: 62 % der Theorie.
Schmelzpunkt: 190-191 °C (Essigester)
$C_{19}H_{20}N_4O_2$ (336,40)
Ber.: C 67.84 H 5.99 N 16.66
Gef.: C 67.83 H 5.98 N 16,80

Beispiel 39

2-(4-Pyridyl)-5,8,8-triäthyl-8-hydro-(5H)-imidazo[5,4-g][3,1]benzoxazin-6-on

Hergestellt analog Beispiel 1 aus 6-Isonicotinoylamido-7-nitro-1,4,4-triäthyl-4H-3.1-benzoxazin-2-on.
Ausbeute: 62 % der Theorie,
Schmelzpunkt: 231-232 °C (Essigester)
$C_{20}H_{22}N_4O_2$ (350,42)
Ber.: C 68,55 H 6,33 N 15,99
Gef.: C 68,47 H 6,43 N 15,93

Beispiel 40

2-(3-Pyridyl)-5,8,8-triäthyl-8-hydro-(5H)-imidazo[5.4-g][3,1]benzoxazin-6-on

Hergestellt analog Beispiel 1 aus 6-Nicotinoylamido-7-nitro-1,4.4-triäthyl-4H-3,1-benzoxazin-2-on.
Ausbeute: 61 % der Theorie.
Schmelzpunkt: 188-189 °C (Essigester)
$C_{20}H_{22}N_4O_2$ (350,42)
Ber.: C 68,55 H 6,33 N 15,99
Gef.: C 68,35 H 6,29 N 15,97

Beispiel 41

2-(4-Methoxy-phenyl)-5,8,8-triäthyl-8-hydro-(5H)-imidazo[5,4-g][3,1]benzoxazin-6-on

Hergestellt analog Beispiel 1 aus 6-(4-Methoxy-benzoylamido)-7-nitro-1,4,4-triäthyl-4H-3,1-benzoxazin-2-on.
Ausbeute: 39 % der Theorie,
Schmelzpunkt: 218-219 °C (Essigester)
$C_{22}H_{25}N_3O_3$ (379,46)
Ber.: C 69,64 H 6,64 N 11,07
Gef.: C 69,49 H 6,68 N 11,15

Beispiel 42

8.8-Dimethyl-2-(1-phenyl-äthyl)-8-hydro-(5H)-imidazo[5,4-g][3,1]benzoxazin-6-on

Hergestellt analog Beispiel 1 aus 4,4-Dimethyl-6-(2-phenylpropionylamido)-7-nitro-4H-3,1-benzoxazin-2-on.
Ausbeute: 58,1 % der Theorie,
Schmelzpunkt: > 280°C (Isopropanol)
$C_{19}H_{19}N_3O_2$ (321,38)
Ber.: C 71,01 H 5,96 N 13,07
Gef.: C 70,99 H 6,10 N 12,97

Beispiel 43

2-(2,4-Dimethoxy-phenyl)-8.8-dimethyl-8-hydro-(5H)-imidazo[5,4-g][3,1]benzoxazin-6-on

4,4 g (0,011 Mol) 6-(2,4-Dimethoxy-benzoylamido)-4.4-dimethyl-7-nitro-4H-3,1-benzoxazin-2-on werden in 100 ml Eisessig gelöst, mit 1 g 10 %igem Palladium/Kohle versetzt und während 15 Minuten bei 70°C und 5 bar Wasserstoffdruck im Autoklaven hydriert. Man saugt vom Katalysator ab. erhitzt das Filtrat noch 2 Stunden auf dem Dampfbad und destilliert das Lösungsmittel bei vermindertem Druck ab. Der Rückstand wird aus Isopropanol/Methanol umkristallisiert.
Ausbeute: 0,65 g (16,7 % der Theorie),
Schmelzpunkt: > 285°C (Zers.)
$C_{19}H_{19}N_3O_4$ (353,38)
Ber.: C 64,58 H 5,42 N 11,89
Gef.: C 64,43 H 5,24 N 11,86

Beispiel 44

2-Benzyl-8,8-dimethyl-8-hydro-(5H)-imidazo[5,4-g][3,1]benzoxazin-6-on

Hergestellt analog Beispiel 43 aus 4,4-Dimethyl-7-nitro-6-phenylacetamido-4H-3,1-benzoxazin-2-on.
Ausbeute: 25,3 % der Theorie,
Schmelzpunkt: 268-270°C (Essigester/Isopropanol)
$C_{18}H_{17}N_3O_2$ (307,35)
Ber.: C 70,34 H 5,57 N 13,67
Gef.: C 70,23 H 5,71 N 13,51

Beispiel 45

8,8-Dimethyl-2-n-propyl-8-hydro-(5H)-imidazo[5,4-g][3,1]benzoxazin-6-on

Hergestellt analog Beispiel 43 aus 6-Butyrylamido-4,4-dimethyl-7-nitro-4H-3,1-benzoxazin-2-on.
Ausbeute: 29 % der Theorie,
Schmelzpunkt: 130-132°C (Essigester/Isopropanol)
$C_{14}H_{17}N_3O_2$ (259,31)
Ber.: C 64,85 H 6,61 N 16,20
Gef.: C 64,74 H 6,81 N 16,10

Beispiel 46

8.8-Dimethyl-2-(1-methyl-butyl)-8-hydro-(5H)-imidazo[5.4-g][3,1]benzoxazin-6-on

Hergestellt analog Beispiel 43 aus 4.4-Dimethyl-6-(2-methyl valerylamido)-7-nitro-4H-3.1-benzoxazin-2-on.
Ausbeute: 18.9 % der Theorie.
Schmelzpunkt: > 280°C (Essigester/Isopropanol)
$C_{16}H_{21}N_3O_2$ (287,36)
Ber.: C 66,88 H 7,37 N 14,62
Gef.: C 67,01 H 7,36 N 14,73

Beispiel 47

8.8-Dimethyl-2-(4-methylsulfonyl-phenyl)-8-hydro-(5H)-imidazo[5,4-g][3,1]benzoxazin-6-on

Hergestellt analog Beispiel 43 aus 4,4-Dimethyl-6-(4-methylsulfonyl-benzoylamido)-7-nitro-4H-3,1-benzoxazin-2-on.
Ausbeute: 52,7 % der Theorie.
Schmelzpunkt: 240°C (Essigester/Isopropanol)
Ber.: C 58.20 H 4.61 N 11,32 S 8.64
Gef.: C 58.15 H 4.82 N 11.36 S 8,40

Beispiel 48

2-(4-Amino-3,5-dibrom-phenyl)-8.8-dimethyl-8-hydro-(5H)-imidazo[5.4-g][3.1]benzoxazin-6-on

5,05 g (0,01 Mol) 6-(4-Amino-3,5-dibrom-benzoylamido)-4,4-dimethyl-7-nitro-4H-3,1-benzoxazin-2-on werden in 100 ml Eisessig gelöst, mit 0,5 g 5 %igem Platin/Kohle versetzt und im Autoklaven bei Raumtemperatur und 5 bar Wasserstoffdruck während einer Stunde hydriert. Nach Absaugen des Katalysators wird das Filtrat noch 4 Stunden auf dem Dampfbad erhitzt, das Lösungsmittel abdestilliert und der Rückstand in Methanol aufgenommen. Durch Zugabe von wässerigem Ammoniak stellt man alkalisch, fällt das Produkt durch Zugabe von Wasser aus, saugt ab, trocknet und kristallisiert aus Isopropanol/Essigester um.
Ausbeute: 1,65 g (36 % der Theorie),
Schmelzpunkt: 235°C
$C_{17}H_{14}Br_2N_4O_2$ (466,16)
Ber.: C 43.80 H 3.03 N 12,02 Br 34,28
Gef.: C 43,72 H 3,16 N 11,90 Br 33,99

Beispiel 49

5-Äthyl-2-(4-amino-3,5-dibrom-phenyl)-8,8-dimethyl-8-hydro-(5H)-imidazo[5,4-g][3,1]benzoxazin-6-on

Hergestellt analog Beispiel 48 aus 1-Äthyl-6-(4-amino-3,5-dibrom-benzoylamido)-4,4-dimethyl-7-nitro-4H-3,1-benzoxazin-2-on.
Ausbeute: 48 % der Theorie,
Schmelzpunkt: > 300°C (Essigester/Isopropanol)
$C_{19}H_{18}Br_2N_4O_2$ (494,21)
Ber.: C 46,18 H 3,67 N 11,34 Br 32,34
Gef.: C 46,00 H 3,89 N 11,09 Br 32,34

Beispiel 50

2-(4-Amino-3,5-dichlor-phenyl)-8,8-dimethyl-8-hydro-(5H)-imidazo[5.4-g][3.1]benzoxazin-6-on x 0,3 $H_2O$

Hergestellt analog Beispiel 48 aus 6-(4-Amino-3.5-dichlor-benzoylamido)-4.4-dimethyl-7-nitro-4H-3.1-benzoxazin-2-on.
Ausbeute: 44 % der Theorie.
Schmelzpunkt: 229°C (Isopropanol/Dioxan)
$C_{17}H_{14}Cl_2N_4O_2$ (382,63)
Ber.: C 53,36 H 3,85 N 14,64 Cl 18,53
Gef.: C 53,23 H 3,96 N 14,49 Cl 18,72

Beispiel 51

5-Äthyl-2-(4-amino-3,5-dichlor-phenyl)-8-hydro-(5H)-imidazo[5,4-g][3,1]benzoxazin-6-on

Hergestellt analog Beispiel 48 aus 1-Äthyl-6-(4-amino-3,5-dichlor-benzoylamido)-4,4-dimethyl-7-nitro-4H-3,1-benzoxazin-2-on.
Ausbeute: 60 % der Theorie,
Schmelzpunkt: 295-297°C (Dioxan/Methanol)
$C_{19}H_{18}Cl_2N_4O_2$ (405,30)
Ber.: C 56,31 H 4.48 N 13,82 Cl 17,50
Gef.: C 56,26 H 4,67 N 13,61 Cl 17,33

Beispiel 52

2-(4-Chinolyl)-8,8-dimethyl-8-hydro-(5H)-imidazo[5,4-g][3,1]benzoxazin-6-on

Hergestellt analog Beispiel 48 aus 6-(4-Chinolin-carbonamido)-4,4-dimethyl-7-nitro-4H-3,1-benzoxazin-2-on.
Ausbeute: 46 % der Theorie,
Schmelzpunkt: 270-272°C (Dioxan/Wasser)
$C_{20}H_{16}N_4O_2$ (344,38)
Ber.: C 69,76 H 4,68 N 16,27
Gef.: C 69,82 H 4,70 N 16,51

Beispiel 53

5-Äthyl-8,8-dimethyl-2-(2-thienyl)-8-hydro-(5H)-imidazo[5,4-g] [3,1]benzoxazin-6-on

Hergestellt analog Beispiel 48 aus 1-Äthyl-4,4-dimethyl-6-(2-thenoylamido)-7-nitro-4H-3,1-benzoxazin-2-on.
Ausbeute: 49 % der Theorie,
Schmelzpunkt: 298-299°C (Dioxan/Wasser)
$C_{17}H_{17}N_3O_2$ (327,41)
Ber.: C 62,36 H 5,23 N 12,83 S 9,79
Gef.: C 62,27 H 5,10 N 13,00 S 9,57

Beispiel 54

5-Äthyl-8,8-dimethyl-2-(2-furyl)-8-hydro-(5H)-imidazo[5,4-g][3,1]benzoxazin-6-on

Hergestellt analog Beispiel 48 aus 1-Äthyl-4,4-dimethyl-6-(2-furoylamido)-7-nitro-4H-3,1-benzoxazin-2-on.
Ausbeute: 33 % der Theorie.
Schmelzpunkt: 300-302°C (Dioxan/Isopropanol/Wasser)
$C_{17}H_{17}N_3O_3$ (311,34)
Ber.: C 65,58 H 5,50 N 13,50
Gef.: C 65,40 H 5,58 N 13,47

Beispiel 55

8,8-Dimethyl-2-(2-furyl)-8-hydro-(5H)-imidazo[5,4-g][3,1]benzoxazin-6-on

Hergestellt analog Beispiel 48 aus 4,4-Dimethyl-6-(2-furoyl amido)-7-nitro-4H-3,1-benzoxazin-2-on.
Ausbeute: 32 % der Theorie,
Schmelzpunkt: 271-272°C (Isopropanol/Essigester)
$C_{15}H_{13}N_3O_3$ (283,29)
Ber.: C 63.60 H 4.63 N 14.83
Gef.: C 63,47 H 4,74 N 14,67

Beispiel 56

8,8-Dimethyl-2-(2-thienyl)-8-hydro (5H)-imidazo[5,4-g][3,1]benzoxazin-6-on $\times$ 0,3 $H_2O$

Hergestellt analog Beispiel 48 aus 4,4-Dimethyl-7-nitro-6-(2-thenoylamido)-4H-3,1-benzoxazin-2-on.
Ausbeute: 16 % der Theorie,
Schmelzpunkt: 275-277°C (Essigester/Isopropanol)
$C_{15}H_{13}N_3O_2$ $\times$ 0,3 $H_2O$ (304,75)
Ber.: C 59,12 H 4,50 N 13,79 S 10,52
Gef.: C 59,23 H 4,63 N 13,90 S 10,38

Beispiel 57

8,8-Dimethyl-2-(2-morpholino-3-pyridyl)-8-hydro-(5H)-imidazo[5,4-g][3,1]benzoxazin-6-on

Hergestellt analog Beispiel 48 aus 4,4-Dimethyl-6-(2-morpholino-nicotinoylamido)-7-nitro-4H-3,1-benzoxazin-2-on.
Ausbeute: 48 % der Theorie,
Schmelzpunkt: 284-285°C (Essigester/Isopropanol)
$C_{20}H_{21}N_5O_3$ (379,42)
Ber.: C 63,31 H 5,58 N 18,46
Gef.: C 63,14 H 5,64 N 18,55

Beispiel 58

8,8-Dimethyl-2-(2-pyrazinyl)-8-hydro-(5H)-imidazo[5,4-g][3,1]benzoxazin-6-on

Hergestellt analog Beispiel 48 aus 4,4-Dimethyl-7-nitro-6-(2-pyrazinoylamido)-4H-3,1-benzoxazin-2-on.
Ausbeute: 17,5 % der Theorie,
Schmelzpunkt: > 300°C (Isopropanol)
$C_{15}H_{13}N_5O_2$ (295,30)
Ber.: C 60,86 H 4,44 N 23,72
Gef.: C 60,86 H 4,62 N 23,95

Beispiel 59

8,8-Dimethyl-2-(4-methylmercapto-phenyl)-8-hydro-(5H)-imidazo[5,4-g][3.1]benzoxazin-6-on × 0,4 $H_2O$

Hergestellt analog Beispiel 48 aus 4,4-Dimethyl-6-(4-methylmercapto-benzoylamido)-7-nitro-4H-3.1-benzoxazin-2-on.
Ausbeute: 30,3 % der Theorie,
Schmelzpunkt: 190-192°C (Essigester)
$C_{19}H_{17}N_3O_2S$ × 0,4 $H_2O$ (346,62)
Ber.: C 62,37 H 5,18 N 12,12 S 9,25
Gef.: C 62,25 H 5,37 N 11,90 S 9,20

Beispiel 60

8,8-Dimethyl-2-[2-(4-pyridyl)-äthyl]-8-hydro-(5H)-imidazo[5,4-g][3,1]benzoxazin-6-on × 0,8 $H_2O$

Hergestellt analog Beispiel 1 aus 4,4-Dimethyl-7-nitro-6-[3-(4-pyridyl)-propionylamido]-4H-3,1-benzoxazin-2-on.
Ausbeute: 59 % der Theorie,
Schmelzpunkt: 127-128°C (Essigester/Isopropanol)
$C_{18}H_{18}N_4O_2$ × 0,8 $H_2O$ (336,78)
Ber.: C 64,20 H 5,87 N 16,64
Gef.: C 64,27 H 6,03 N 16,42

Beispiel 61

8,8-Dimethyl-2-(4-pyridyl)-8-hydro-(5H)-imidazo[5,4-g][3,1]benzoxazin-6-on

Zu einer Suspension von 2,07 g (0,01 Mol) 6,7-Diamino-4,4-dimethyl-4H-3,1-benzoxazin-2-on und 2,67 g (0,015 Mol) Isonicotinsäurechlorid-hydrochlorid in 100 ml Methylenchlorid werden bei Raumtemperatur 5 g (0,05 Mol) Triethylamin zugetropft. Nach einstündigem Rühren bei Raumtemperatur wird das Lösungsmittel abdestilliert, der Rückstand mit 50 ml Äthanol, 25 ml konzentrierter Salzsäure und 10 ml Wasser versetzt und 18 Stunden zum Rückfluß erhitzt. Der nach Abdestillieren der Lösungsmittel verbleibende Rückstand wird in Äthanol aufgenommen, mit Ammoniak alkalisch gestellt und langsam mit Wasser versetzt. Der dabei ausfallende Niederschlag wird abgesaugt und aus Dioxan/Wasser umkristallisiert.
Ausbeute: 1,2 g (40,8 % der Theorie),
Schmelzpunkt: > 300°C

Beispiel 62

8,8-Dimethyl-5-(2-methoxy-äthyl)-2-(2-pyrazinyl)-8-hydro-(5H)-imidazo[5,4-g][3,1]benzoxazin-6-on

Hergestellt analog Beispiel 1 aus 4,4-Dimethyl-1-(2-methoxy-äthyl)-7-nitro-6-(2-pyrazinoylamido)-4H-3,1-benzoxazin-2-on.
Ausbeute: 46,6 % der Theorie,
Schmelzpunkt: 227-228°C (Essigester/Isopropanol)
$C_{18}H_{19}N_5O_3$ (353,38)
Ber.: C 61,18 H 5,42 N 19,82
Gef.: C 61,14 H 5,48 N 19,64

Beispiel 63

8.8-Dimethyl-2-(4-nitro-phenyl)-8-hydro-(5H)-imidazo[5.4-g][3.1]benzoxazin-6-on

Hergestellt analog Beispiel 61 aus 6.7-Diamino-4.4-dimethyl-4H-3.1-benzoxazin-2-on und 4-Nitro-benzoylchlorid.
Ausbeute: 22 % der Theorie.
Schmelzpunkt: > 300°C (Dioxan/Isopropanol)
$C_{17}H_{14}N_4O_4$ (338,32)
Ber.: C 60,35 H 4,17 N 16,56
Gef.: C 60,10 H 4,33 N 16,38

Beispiel 64

8.8-Dimethyl-2-(4-pyridyl)-8-hydro-(5H)-imidazo[5.4-g][3.1]benzoxazin-6-on

2,07 g (0,01 Mol) 6,7-Diamino-4,4-dimethyl-4H-3,1-benzoxazin-2-on werden in 40 ml Äthanol und 2 ml Eisessig gelöst und mit 1,07 g (0,01 Mol) Pyridin-4-aldehyd versetzt. Die Mischung wird 1 Stunde zum Rückfluß erhitzt und 2 weitere Stunden unter Lufteinleitung. Danach wird das Lösungsmittel abdestilliert, der Rückstand in Äthanol aufgeschlämmt, mit Ammoniak alkalisch gestellt und langsam mit Wasser versetzt. Der anfallende Niederschlag wird abgesaugt und aus Dioxan/Wasser umkristallisiert.
Ausbeute: 1,8 g (61,2 % der Theorie),
Schmelzpunkt: > 300°C

Beispiel 65

8.8-Dimethyl-2-(4-methylsulfinyl-phenyl)-8-hydro-(5H)-imidazo[5,4-g][3,1]benzoxazin-6-on x 0,5 $H_2O$

0,69 g (0,002 Mol) 8,8-Dimethyl-2-(4-methylmercapto-phenyl)-8-hydro-(5H)-imidazo[5,4-g][3,1]-benzoxazin-6-on werden in 40 ml Eisessig gelöst, mit 0,2 ml 30%igem Wasserstoffperoxid versetzt und 16 Stunden bei Raumtemperatur stehen gelassen. Der nach Abziehen des Lösungsmittels verbleibende Rückstand wird in Isopropanol/Diisopropyläther umkristallisiert.
Ausbeute: 0,42 g (58 % der Theorie),
Schmelzpunkt: 212-214°C
$C_{18}H_{17}N_3O_3S$ x 0,5 $H_2O$ (364,42)
Ber.: C 59,32 H 4,98 N 11,53 S 8,80
Gef.: C 59,13 H 5,14 N 11,60 S 9,01

Beispiel 66

2-(2-Phenyl-äthyl)-5.8.8-trimethyl-8-hydro-(5H)-imidazo[5.4-g][3.1]benzoxazin-6-on

0,66 g (0,002 Mol) 2-(2-Phenyl-vinyl)-5,8,8-trimethyl-8-hydro-(5H)-imidazo[5,4-g][3,1]benzoxazin-6-on werden in 20 ml Methanol gelöst, mit 0,1 g 10%iger Palladium/Kohle versetzt und im Autoklaven bei Raumtemperatur und 5 bar Wasserstoff druck während 1 Stunde hydriert. Nach Abfiltrieren des Katalysators wird das Lösungsmittel abdestilliert, der Rückstand in Wasser aufgerührt, abgesaugt, getrocknet und aus Essigester umkristallisiert.
Ausbeute: 0,5 g (74,3 % der Theorie),
Schmelzpunkt: 194-195°C
$C_{20}H_{21}N_3O_2$ x 0,25 $H_2O$ (339,91)
Ber.: C 70,67 H 6,38 N 12,36
Gef.: C 70,70 H 6,32 N 12,32

Beispiel 67

2-(4-Hydroxy-phenyl)-5,8,8-trimethyl-8-hydro-(5H)-imidazo[5,4-g][3,1]benzoxazin-6-on

4,75 g (0,014 Mol) 2-(4-Methoxy-phenyl)-5,8,8-trimethyl-8-hydro-(5H)-imidazo[5,4-g][3,1]benzoxazin-6-on werden in 250 ml Äthylenchlorid gelöst, bei -30°C mit 12,5 g Bortribromid versetzt und 10 Tage bei Raumtemperatur gerührt. Nach vorsichtiger Zugabe von 50 ml Äthanol/Wasser (1:1) werden die Lösungsmittel abdestilliert, der Rückstand in Wasser aufgerührt, abgesaugt und zweimal aus Dimethylformamid/Wasser umkristallisiert.
Ausbeute: 1,1 g (22,2 % der Theorie),
Schmelzpunkt: ab 270°C (Zers.)
$C_{18}H_{17}N_3O_3 \times 1,6\ H_2O$ (352,18)
Ber.: C 61,39 H 5,78 N 11,93
Gef.: C 61,15 H 5,62 N 11,73

Beispiel 68

2-(2-Phenyl-vinyl)-5,8,8-trimethyl-8-hydro-(5H)-imidazo[5,4-g][3,1]benzoxazin-6-on

2,15 g (0,0061 Mol) 7-Amino-6-cinnamoylamido-1,4,4-trimethyl-4H-3,1-benzoxazin-2-on (hergestellt aus 6-Cinnamoylamido-7-nitro-1,4,4-trimethyl-4H-3,1-benzoxazin-2-on und Eisen/konzentrierte Salzsäure in Isopropanol) werden in 20 ml Eisessig gelöst, 4 Stunden auf dem Dampfbad erhitzt und das Lösungsmittel abdestilliert. Der Rückstand wird in Methanol/Wasser gelöst und durch Zugabe von Ammoniak alkalisch gestellt. Der bei Zugabe von weiterem Wasser ausfallende Niederschlag wird abgesaugt, getrocknet und aus Essigester/Isopropanol umkristallisiert.
Ausbeute: 1,2 g (58,8 % der Theorie),
Schmelzpunkt: 249-250°C
$C_{20}H_{19}N_3O_2$ (333,39)
Ber.: C 72,05 H 5,74 N 12,60
Gef.: C 72,14 H 6,01 N 12,44

Beispiel 69

8,8-Dimethyl-5-isopropyl-2-(4-methoxy-phenyl)-8-hydro-(5H)-imidazo[5,4-g][3,1]benzoxazin-6-on × 3 $H_2O$

Hergestellt analog Beispiel 68 aus 7-Amino-4,4-dimethyl-1-isopropyl-6-(4-methoxy-benzoylamido)-4H-3,1-benzoxazin-2-on und Eisessig/Salzsäure.
Ausbeute: 81,1 % der Theorie,
Schmelzpunkt: 291-293°C (Eisessig/Wasser = 1:1)
$C_{21}H_{23}N_3O_3 \times 3\ H_2O$ (419,48)
Ber.: C 60,13 H 6,97 N 10,02
Gef.: C 60,08 H 6,78 N 10,10

Beispiel 70

2-(4-Cyan-phenyl)-8,8-dimethyl-8-hydro-(5H)-imidazo[5,4-g][3,1]benzoxazin-6-on × 0,6 $H_2O$

Hergestellt analog Beispiel 68 aus 7-Amino-6-(4-cyan-benzoylamido)-4,4-dimethyl-4H-3,1-benzoxazin-2-on und Eisessig. Ausbeute: 16 % der Theorie,
Schmelzpunkt: 225°C (Essigester/Isopropanol)
$C_{18}H_{14}N_4O_2 \times 0,6\ H_2O$ (329,14)
Ber.: C 65,69 H 4,65 N 17,02
Gef.: C 65,78 H 4,81 N 16,88

Beispiel 71

2-(4-Piperidinyl)-5,8,8-trimethyl-8-hydro-(5H)-imidazo[5,4-g][3,1]benzoxazin-6-on × 1,5 $H_2O$

1,5 g (0,004 Mol) 2-(4-Pyridyl)-5,8,8-trimethyl-8-hydro-(5H)-imidazo[5,4-g][3,1]benzoxazin-6-on-tetrahydrat werden in 150 ml Eisessig gelöst, mit 0,3 g Platinoxid versetzt und im Autoklaven während 3 Stunden bei Raumtemperatur und 3 bar Wasserstoffdruck hydriert. Nach Abfiltrieren des Katalysators destilliert man das Lösungsmittel ab, versetzt mit gesättigter Kaliumcarbonat-Lösung und extrahiert mehrmals mit Essigester. Die Essigester-Phase wird mit gesättigter Kochsalz-Lösung gewaschen, getrocknet und auf etwa ein Viertel des Volumens eingeengt. Bei Zugabe von Äther bildet sich ein Niederschlag, den man absaugt und trocknet.

Ausbeute: 0,65 g (48 % der Theorie),
Schmelzpunkt: 235°C (Essigester/Äther)
$C_{17}H_{22}N_4O_2$ × 1,5 $H_2O$ (341,41)
Ber.: C 59,81 H 7,38 N 16,41
Gef.: C 59,85 H 7,66 N 16,14

Beispiel 72

8,8-Dimethyl-2-mercapto-8-hydro-(5H)-imidazo[5,4-g][3,1]benzoxazin-6-on

Eine Mischung von 100 ml Wasser, 50 ml Äthanol, 50 ml Schwefelkohlenstoff, 11,2 g (0,02 Mol) Kaliumhydroxid und 20,7 g (0,1 Mol) 6,7-Diamino-4,4-dimethyl-4H-3,1-benzoxazin-2-on wird unter Rühren 5 Stunden lang zum Rückfluß erhitzt. Nach dem Abkühlen wird durch Zugabe von ca. 20 ml Eisessig angesäuert, der Niederschlag abgesaugt und aus Dimethylformamid/Wasser umkristallisiert.
Ausbeute: 15,6 g (62,6 % der Theorie),
Schmelzpunkt: ab 293°C (Zers.)
$C_{11}H_{11}N_3O_2S$ (249,30)
Ber.: C 53,00 H 4,45 N 16,86 S 12,86
Gef.: C 52,88 H 4,50 N 17,00 S 13,00

Beispiel 73

8,8-Dimethyl-2-methylmercapto-8-hydro-(5H)-imidazo[5,4-g][3,1]benzoxazin-6-on

Zu einer aus 0.23 g (0.01 Mol) Natrium und 50 ml absolutem Äthanol hergestellten Natriumäthylat-Lösung gibt man 2,49 g (0,01 Mol) 8,8-Dimethyl-2-mercapto-8-hydro-(5H)-imidazo[5,4-g][3,1]benzoxazin-6-on und rührt 10 Minuten nach und nach Zugabe von 0,7 ml Methyljodid nochmals 20 Minuten. Die beim Versetzen mit Wasser ausfallenden Kristalle werden abgesaugt, getrocknet und aus Äthylenchlorid/Diisopropyläther umkristallisiert.
Ausbeute: 2 g (76 % der Theorie),
Schmelzpunkt: 273°C
$C_{12}H_{13}N_3O_2S$ (263,31)
Ber.: C 54,74 H 4,98 N 15,96 S 12,18
Gef.: C 55,00 H 4,89 N 15,67 S 11,95

Beispiel 74

8,8-Dimethyl-2-(3-furyl)-8-hydro-(5H)-imidazo[5,4-g][3,1]benzoxazin-6-on

Hergestellt analog Beispiel 1 aus 4,4-Dimethyl-6-(3-furoylamido)-7-nitro-4H-3,1-benzoxazin-2-on.
Ausbeute: 48 % der Theorie,
Schmelzpunkt: 295-296°C (Essigester/Isopropanol)
$C_{15}H_{13}N_3O_3$ (283,29)
Ber.: C 63,60 H 4,63 N 14,83
Gef.: C 63,46 H 4,69 N 14,66

Beispiel 75

5-Äthyl-8,8-dimethyl-2-(3-furyl)-8-hydro-(5H)-imidazo[5,4-g][3,1]benzoxazin-6-on

Hergestellt analog Beispiel 1 aus 1-Äthyl-4,4-dimethyl-6-(3-furoylamido)-7-nitro-4H-3,1-benzoxazin-2-on.
Ausbeute: 43 % der Theorie,
Schmelzpunkt: 288-289°C (Essigester/Isopropanol)
$C_{17}H_{17}N_3O_3$ (311,34)
Ber.: C 65,58 H 5,50 N 13,50
Gef.: C 65.36 H 5,57 N 13,34

Beispiel 76

8,8-Dimethyl-2-(3-thienyl)-8-hydro-(5H)-imidazo[5,4-g][3,1]benzoxazin-6-on

Hergestellt analog Beispiel 1 aus 4,4-Dimethyl-7-nitro-6-(3-thenoylamido)-4H-3,1-benzoxazin-2-on.
Ausbeute: 62 % der Theorie,
Schmelzpunkt: 286-287°C (Essigester/Isopropanol)
$C_{15}H_{13}N_3O_2S$ (299,36)
Ber.: C 60,18 H 4,38 N 14,04 S 10,71
Gef.: C 60,15 H 4,52 N 13,82 S 10,55

Beispiel 77

5-Äthyl-8,8-dimethyl-2-(3-thienyl)-8-hydro-(5H)-imidazo[5,4-g][3,1]benzoxazin-6-on

Hergestellt analog Beispiel 1 aus 1-Äthyl-4,4-dimethyl-7-nitro-6-(3-thenoylamido)-4H-3,1-benzoxazin-2-on.
Ausbeute: 50 % der Theorie,
Schmelzpunkt: 298-299°C (Essigester/Isopropanol)
$C_{17}H_{17}N_3O_2S$ (327,40)
Ber.: C 62,36 H 5,23 N 12,83 S 9,79
Gef.: C 62,17 H 5,21 N 12,69 S 9,99

Beispiel 78

5-Äthyl-8,8-dimethyl-2-(2-pyrazinyl)-8-hydro-(5H)-imidazo[5,4-g][3,1]benzoxazin-6-on

Hergestellt analog Beispiel 1 aus 1-Äthyl-4,4-dimethyl-7-nitro-6-(2-pyrazinoylamido)-4H-3,1-benzoxazin-2-on.
Ausbeute: 42,5 % der Theorie,
Schmelzpunkt: 259°C (Zers.; Essigester/Diisopropyläther)
$C_{17}H_{17}N_5O_2$ (323,35)
Ber.: C 63,15 H 5,30 N 21,66
Gef.: C 62,95 H 5,40 N 21,40

Beispiel 79

8.8-Dimethyl-5-isopropyl-2-(2-pyrazinyl)-8-hydro-(5H)-imidazo[5,4-g][3,1]benzoxazin-6-on × 0,2 $H_2O$

Hergestellt analog Beispiel 1 aus 4,4-Dimethyl-1-isopropyl-7-nitro-6-(2-pyrazinoylamido)-4H-3,1-benzoxazin-2-on.
Ausbeute: 40.2 % der Theorie,
Schmelzpunkt: 298°C (Zers.; Essigester/Diisopropyläther)
$C_{18}H_{19}N_5O_2$ × $0,2H_2O$ (340,98)
Ber.: C 63,40 H 5,73 N 20,54
Gef.: C 63,59 H 5,73 N 20,42

Beispiel 80

2-(2-Pyrazinyl)-5,8,8-trimethyl-8-hydro-(5H)-imidazo[5,4-g][3,1]benzoxazin-6-on

Hergestellt analog Beispiel 1 aus 7-Nitro-6-(2-pyrazinoylamido)-1,4,4-trimethyl-4H-3,1-benzoxazin-2-on.
Ausbeute: 42 % der Theorie,
Schmelzpunkt: 255-256°C (Zers.; Essigester/Diisopropyläther)
$C_{16}H_{15}N_5O_2$ (309,33)
Ber.: C 62.13 H 4.89 N 22,64
Gef.: C 61.93 H 4.94 N 22,70

Beispiel 81

8.8-Dimethyl-5-n-propyl-2-(2-pyrazinyl)-8-hydro-(5H)-imidazo[5,4-g][3,1]benzoxazin-6-on

Hergestellt analog Beispiel 1 aus 4,4-Dimethyl-7-nitro-1-n-propyl-6-(2-pyrazinoylamido)-4H-3,1-benzoxazin-2-on.
Ausbeute: 47,3 % der Theorie,
Schmelzpunkt: 270-271°C (Essigester/Isopropanol)
$C_{18}H_{19}N_5O_2$ (337,38)
Ber.: C 64,08 H 5,68 N 20,76
Gef.: C 63,94 H 5,69 N 20,51

Beispiel 82

5-n-Butyl-8,8-dimethyl-2-(2-pyrazinyl)-8-hydro-(5H)-imidazo[5,4-g][3,1]benzoxazin-6-on

Hergestellt analog Beispiel 1 aus 1-n-Butyl-4,4-dimethyl-7-nitro-6-(2-pyrazinoylamido)-4H-3,1-benzoxazin-2-on.
Ausbeute: 37,6 % der Theorie,
Schmelzpunkt: 216-217°C
$C_{19}H_{21}N_5O_2$ (351,41)
Ber.: C 64,94 H 6,02 N 19,93
Gef.: C 64,84 H 6,11 N 19,77

Beispiel I

Dragées mit 5 mg 8.8-Dimethyl-2-(4-pyridyl)-8-hydro-(5H)-imidazo[5,4-g][3.1]benzoxazin-6-on

1 Dragéekern enthält:

| | |
|---|---|
| Wirksubstanz | 5,0 mg |
| Milchzucker | 25,2 mg |
| Maisstärke | 18,0 mg |
| Cellulose, mikrokristallin | 10,0 mg |
| Polyvinylpyrrolidon | 1,0 mg |
| Magnesiumstearat | 0,8 mg |
| | 60,0 mg |

Herstellverfahren:

Der Wirkstoff, Milchzucker, Maisstärke, Cellulose und Polyvinylpyrrolidon werden gemischt und mit Wasser granuliert. Das Granulat wird bei einer Temperatur von 45° C getrocknet und die angegebene Menge Magnesiumstearat zugemischt. Auf einer Tablettiermaschine werden bikonvexe Kerne mit einem Durchmesser von 5 mm hergestellt.

Dragierung:

Die Kerne werden mit einer 15 %igen Polyvinylpyrrolidon-Lösung überzogen und mit Dragiersuspension bis zu einem Gewicht von 80 mg fertigdragiert.

Beispiel II

Tabletten mit Teilkerbe zu 50 mg 8,8-Dimethyl-2-(4-pyridyl)-8-hydro-(5H)-imidazo[5,4-g][3,1]benzoxazin-6-on

Zusammensetzung:

1 Tablette enthält:

| | |
|---|---|
| Wirksubstanz | 50,0 mg |
| Milchzucker | 40,0 mg |
| Maisstärke | 17,0 mg |
| Polyvinylpyrrolidon | 2,0 mg |
| Magnesiumstearat | 1,0 mg |
| | 110,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50° C wird erneut gesiebt (1,5 mm-Maschenweite) und das Schmiermittel zugemischt.

Die preßfertige Mischung wird zu Tabletten mit Teilkerbe verarbeitet.

Beispiel III

Suppositorien mit 100 mg 8,8-Dimethyl-2-(4-pyridyl)-8-hydro-(5H)-imidazo[5.4-g][3,1]benzoxazin-6-on

1 Zäpfchen enthält:

| | |
|---|---|
| Wirksubstanz | 100,0 mg |
| Suppositorienmassen (Hartfett) | 1600,0 mg |
| | 1700,0 mg |

Herstellungsverfahren:

Die feinpulverisierte Wirksubstanz wird in die geschmolzene und auf 40° C abgekühlte Zäpfchenmasse einhomogenisiert. Man kühlt auf 37° C ab und gießt die Masse in leicht vorgekühlte Formen aus. Zäpfchengewicht: 1,7 g.

Beispiel IV

Ampullen zu 10 mg 8,8-Dimethyl-2-(4-pyridyl)-8-hydro-(5H)-imidazo[5,4-g][3,1]benzoxazin-6-on

Zusammensetzung: Wirksubstanz 10,0 mg
0,1 n HCl 0,34 ml
Aqua bidest. ad 1,0 ml

Herstellung:

Die Substanz wird in Wasser unter Zusatz von Salzsäure gelöst, unter aseptischen Bedingungen durch einen Membranfilter filtriert und anschließend in braune, gereinigte und sterilisierte 2 ml-Injektionsfläschchen abgefüllt.

**Ansprüche**

1. Neue Imidazo-benzoxazinone der Formel

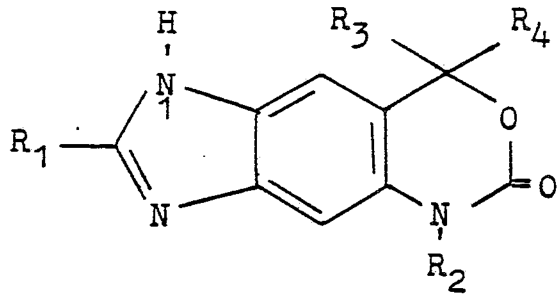

,(I)

in der

R· eine gegebenenfalls durch eine Phenyl-oder Pyridylgruppe substituierte Alkylgruppe, eine Alkylgruppe mit 4 bis 6 Kohlenstoffatomen, eine gegebenenfalls durch eine Alkylgruppe substituierte Mercaptogruppe, eine Vinylengruppe, die endständig durch eine Phenyl-oder Pyridylgruppe substituiert ist, eine gegebenenfalls durch ein Halogenatom, eine Hydroxy-, Alkoxy-, Mercapto-, Alkylmercapto-, Alkylsulfinyl-, Alkylsulfonyl-, Cyano-, Amino-oder Nitrogruppe substituierte Phenylgruppe, wobei eine Hydroxyphenylgruppe zusätzlich durch eine oder zwei Alkylgruppen mit jeweils 1 bis 4 Kohlenstoffatomen oder eine Aminophenylgruppe durch ein oder zwei Halogenatome substituiert sein kann, eine durch Halogenatome, Hydroxy-, Alkoxy-, Mercapto-, Alkylmercapto-, Alkylsulfinyl-oder Alkylsulfonylgruppen disubstituierte Phenylgruppe, wobei die Substituenten des Phenylkerns gleich oder verschieden sein können, einen über ein Kohlenstoffatom gebundenen 5-oder 6-gliedrigen heteroaromatischen Ring, welcher ein Sauerstoff-, Schwefel-oder Stickstoffatom, zwei oder drei Stickstoffatome oder ein Stickstoffatom und ein Sauerstoff-oder Schwefelatom enthält, an den zusätzlich über zwei benachbarte Kohlenstoffatome eine oder zwei 1,4-Butadienylgruppen gebunden sein können, wobei im Falle der Pyridine diese zusätzlich durch eine Amino-, Alkanoylamino-oder Morpholinogruppe oder durch zwei Halogenatome oder durch ein Halogenatom und eine Amino-oder Morpholinogruppe substituiert sein können, oder einen über einen Kohlenstoffatom gebundenen 5-bis 7-gliedrigen gesättigten Imino-, N-Alkyl-imino-oder N-Alkanoyl-imino-alkylenring in dem eine Methylengruppe in 4-Stellung durch eine Imino-, Alkylimino-oder Alkanoyliminogruppe oder eine $-CH_2CH_2-$Gruppe durch eine -NH-CO-Gruppe ersetzt sein kann, wobei die CO-Gruppe dieser -NH-CO-Gruppe mit dem bereits vorhandenen N-Atom verknüpft sein muß, und außerdem alle vorstehend erwähnten heteroaromatischen Ringe und gesättigten Ringe durch eine Alkylgruppe substituiert sein können,

$R_2$ ein Wasserstoffatom, eine gegebenenfalls ab Position 2 durch eine Hydroxy-oder Alkoxygruppe substituierte Alkylgruppe mit 1 bis 6 Kohlenstoffatomen oder eine Phenylalkylgruppe,

$R_3$ und $R_4$, die gleich oder verschieden sein können, Wasserstoffatome oder Alkylgruppe, wobei der Alkylteil aller vorstehend erwähnten Gruppen, sofern nichts anderes erwähnt wird, jeweils 1 bis 3 Kohlenstoffatome enthalten kann, bedeuten, deren 3H-Tautomere und deren Säureadditionssalze.

2. Neue Imidazo-benzoxazinone der Formel I gemäß Anspruch 1, in der

$R_1$ eine gegebenenfalls durch eine Phenyl-oder Pyridylgruppe substituierte Alkylgruppe mit 1 oder 2 Kohlenstoffatomen, eine Alkylgruppe mit 3 bis 5 Kohlenstoffatomen, eine gegebenenfalls durch eine Methylgruppe substituierte Mercaptogruppe, eine Vinylengruppe, die endständig durch eine Phenyl-oder Pyridylgruppe substituiert ist, eine gegebenenfalls durch eine Hydroxy-, Methoxy-, Methylmercapto-, Methyl sulfinyl-, Methylsulfonyl-, Cyano-oder Nitrogruppe substituierte Phenylgruppe, eine Dimethoxy-phenyl-, 3,5-Di-tert.butyl-4-hydroxy-phenyl-, 4-Amino-3,5-dihalogen-phenyl-, Pyridyl-, Piperidinyl-, Morpholino-pyridyl-, Chinolyl-, Furanyl-, Thienyl-oder Pyrazinylgruppe,

$R_2$ ein Wasserstoffatom, eine Methyl-, Benzyl-, Äthyl-, n-Propyl-, Isopropyl-, 2-Hydroxy-äthyl-oder 2-Methoxy-äthylgruppe,

$R_3$ und $R_4$, die gleich oder verschieden sein können, Wasserstoffatome, Methyl-oder Ethylgruppen bedeuten, deren 3H-Tautomere und deren Säureadditionssalze.

3. Neue Imidazo-benzoxazinone der Formel I gemäß Anspruch 1, in der

$R_1$ eine Alkylgruppe mit 1 bis 3 Kohlenstoffatomen oder eine Pyridyl-, Pyrazinyl-, Furyl-oder Thienylgruppe,

$R_2$ ein Wasserstoffatom, eine Methyl-oder Ethylgruppe,

$R_3$ und $R_4$ jeweils eine Methylgruppe bedeuten, 3H-Tautomere und deren Säureadditionssalze.

4. 8,8-Dimethyl-2-(4-pyridyl)-8-hydro-(5H)-imidazo[5,4-g][3,1]benzoxazin-6-on, dessen 3H-Tautomeres und dessen Säureadditionssalze.

5. 5-Äthyl-8,8-dimethyl-2-(4-pyridyl)-8-hydro-(5H)-imidazo[5,4-g][3,1]-benzoxazin-6-on, dessen 3H-Tautomeres und dessen Säureadditionssalze.

6. Physiologisch verträgliche Säureadditionssalze der Verbindungen gemäß den Ansprüchen 1 bis 5 mit anorganischen oder organischen Säuren.

7. Arzneimittel, enthaltend eine Verbindung der Formel I gemäß den Ansprüchen 1 bis 5 oder dessen physiologisch verträgliches Säureadditionssalz gemäß Anspruch 6 neben einem oder mehreren inerten Trägerstoffen und/oder Verdünnungsmitteln.

8. Verwendung einer Verbindung gemäß den Ansprüchen 1 bis 5 oder deren physiologisch verträgliche Säureadditionssalze gemäß Anspruch 6 zur Herstellung eines Arzneimittels gemäß Anspruch 7.

9. Verfahren zur Herstellung eines Arzneimittels gemäß Anspruch 8, dadurch gekennzeichnet, daß auf nichtchemischem Wege eine Verbindung gemäß den Ansprüchen 1 bis 5 oder dessen physiologisch verträgliches Säureadditionssalz gemäß Anspruch 6 in einen oder mehreren inerten Trägerstoffen und/oder Verdünnungsmitteln eingearbeitet wird.

10. Verfahren zur Herstellung von neuen Imidazo-benzoxazinonen gemäß den Ansprüchen 1 bis 6, dadurch gekennzeichnet, daß

a) eine Verbindung der allgemeinen Formel

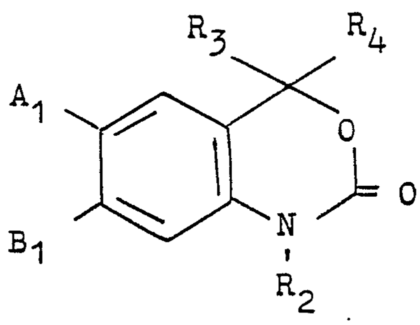

,(II)

in der

$R_2$ bis $R_4$ wie in den Ansprüchen 1 bis 5 definiert sind,

einer der Reste $A_1$ oder $B_1$ eine $NH_2$-Gruppe und der andere der Reste $A_1$ oder $B_1$ eine

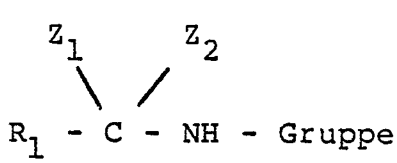

bedeuten, wobei

$R_1$ wie in den Ansprüchen 1 bis 5 definiert ist,

$Z_1$ und $Z_2$, die gleich oder verschieden sein können, nukleophile Austrittsgruppen wie gegebenenfalls substituierte Amino-, Alkoxy-, Phenylalkoxy-, Phenoxy-, Alkylmercapto-, Phenylalkylmercapto-oder Phenylthiogruppen oder

$Z_1$ und $Z_2$ zusammen ein Sauerstoff-oder Schwefelatom, eine gegebenenfalls durch eine Alkyl-oder Phenylalkylgruppe substituierte Iminogruppe, wobei die vorstehend erwähnten Alkylteile jeweils 1 bis 3

Kohlenstoffatome enthalten können, oder eine Alkylendioxigruppe mit 2 oder 3 Kohlenstoffatomen bedeuten, cyclisiert wird oder

b) eine Verbindung der Formel

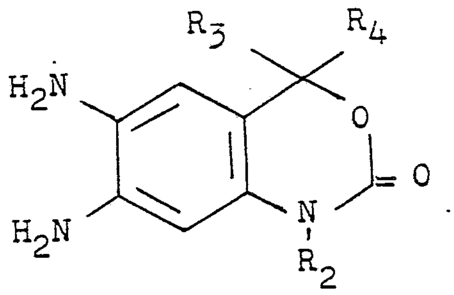

, (IV)

in der

$R_2$, $R_3$ und $R_4$ wie in den Ansprüchen 1 bis 5 definiert sind, mit einem Aldehyd der Formel

$R_1$-CHO ,(VI)

in der

$R_1$ wie in den Ansprüchen 1 bis 5 definiert ist, umgesetzt und anschließend eine so erhaltene Verbindung oxidiert wird und

gewünschtenfalls anschließend eine so erhaltene Verbindung der Formel I, in der $R_2$ ein Wasserstoffatom darstellt und/oder $R_1$ eine Hydroxy-oder Mercaptogruppe enthält, mittels Alkylierung in die entsprechende Alkylverbindung übergeführt wird und/oder

eine so erhaltene Verbindung der Formel I, in der $R_1$ eine endständig durch eine Phenyl-oder Pyridylgruppe substituierte Vinylengruppe darstellt, mittels katalytischer Hydrierung in eine entsprechende Verbindung der Formel I, in der $R_1$ eine endständig durch eine Phenyl-oder Pyridylgruppe substituierte Ethylengruppe darstellt, übergeführt wird und/oder

eine so erhaltene Verbindung der Formel I, in der $R_2$ eine Benzylgruppe darstellt, mittels katalytischer Hydrierung in eine entsprechende Verbindung der Formel I, in der $R_2$ ein Wasserstoff darstellt, übergeführt wird und/oder

eine so erhaltene Verbindung der Formel I, in der $R_1$ ein Pyridinring darstellt, mittels katalytischer Hydrierung in eine entsprechende Verbindung der Formel I, in der $R_1$ eine Piperidinylgruppe darstellt, übergeführt wird und/oder

eine so erhaltene Verbindung der Formel I, in der $R_1$ eine Alkoxyphenyl-oder Dialkoxyphenylgruppe darstellt, mittels Etherspaltung in eine entsprechende Verbindung der Formel I, in der $R_1$ eine Hydroxyphenyl-oder Dihydroxyphenylgruppe darstellt, übergeführt wird und/oder

eine so erhaltene Verbindung der Formel I, in der $R_1$ eine Alkylmercaptophenyl-oder Dialkylmercaptophenylgruppe darstellt, mittels Oxidation in eine entsprechende Verbindung der Formel I, in der $R_1$ eine Alkylsulfinyl-, Alkylsulfonyl-, Dialkylsulfinyl-oder Dialkylsulfonyl-phenylgruppe darstellt, übergeführt wird und/oder

eine so erhaltene Verbindung der Formel I in ihre Säureadditionssalze, insbesondere in ihre physiologisch verträglichen Salze mit anorganischen oder organischen Säuren, übergeführt wird.

Europäisches Patentamt

# EUROPÄISCHER RECHERCHENBERICHT

Nummer der Anmeldung

EP 87113863.2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | THE JOURNAL OF ORGANIC CHEMISTRY, Band 51, No. 5, 7. März 1986<br>STEVENSON et al. "Defined dimensional alterations in enzyme substrates"<br>Seiten 616-620<br>* Seiten 618, 620; Verbindung 19 *<br>-- | 1-5,10 | C 07 D 498/04<br>A 61 K 31/535 |
| A | US - A - 3 883 521 (FAURAN et al.)<br>* Zusammenfassung *<br>-- | 1,7-10 | |
| A | DE - A - 2 227 489 (DELALANDE S.A.)<br>* Seiten 2,3; Ansprüche *<br>-- | 1,7-10 | RECHERCHIERTE SACHGEBIETE (Int. Cl.4) |
| A | DE - A1 - 2 349 480 (BAYER AG)<br>* Ansprüche *<br>---- | 1,10 | C 07 D 498/00 |

Der vorliegende Recherchenbericht wurde für alle Patentansprüche erstellt

| Recherchenort | Abschlußdatum der Recherche | Prüfer |
|---|---|---|
| WIEN | 07-12-1987 | JANISCH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82